# NOUVEAU FASCICULE DE BREVET EUROPEEN

(11) **EP 0 999 823 B2**
(45) Date de publication et mention de la décision concernant l'opposition: **05.10.2011**
(45) Mention de la délivrance du brevet: 16.06.2004
(21) Numéro de dépôt: 98947623.9
(22) Date de dépôt: 07.10.1998
(51) Int. Cl.: A61Q 5/06, A61K 8/49

(54) **COMPOSITION DE TEINTURE DES FIBRES KERATINIQUES ET PROCEDE DE TEINTURE METTANT EN OEUVRE CETTE COMPOSITION**
FÄRBEMITTEL FÜR KERATINISCHE FASER UND VERFAHREN ZUM FÄRBEN UNTER VERWENDUNG DIESER ZUSAMMENSETZUNG
COMPOSITION FOR DYEING KERATIN FIBRES AND DYEING METHOD USING SAME

(30) Priorité: 22.10.1997 FR 9713240
(43) Date de publication de la demande: 17.05.2000
(60) Demande divisionnaire: 04290396.3
(73) Titulaire: L'Oréal, 75008 Paris (FR)
(72) Inventeur: RONDEAU, Christine, F-78500 Sartrouville (FR)
(74) Mandataire: Dossmann, Gérard
(86) Numéro de dépôt international: PCT/FR1998/002145
(87) Numéro de publication internationale: WO 1999/020235

(56) Documents cités:
- WO-A1-95/01772
- WO-A1-95/15144
- DE-A1- 3 131 409
- DE-A1- 3 423 349
- DE-U1- 29 607 212
- US-A- 3 985 499
- US-A- 4 025 301

## Description

L'invention a pour objet une composition pour la teinture des fibres kératiniques, et en particulier des fibres kératiniques humaines telles que les cheveux comprenant, dans un milieu approprié pour la teinture, au moins un colorant direct cationique convenablement sélectionné, et au moins un colorant direct nitré benzénique, ainsi que le procédé de teinture mettant en oeuvre cette composition.

Il est bien connu de teindre les fibres kératiniques et en particulier les cheveux humains avec des compositions tinctoriales contenant des colorants directs et en particulier des colorants directs nitrés benzéniques. Les colorants directs ont cependant l'inconvénient, lorsqu'ils sont incorporés dans des compositions tinctoriales, de conduire à des colorations présentant une ténacité insuffisante, en particulier vis-à-vis des shampooings.

Le document brevet US-A-3 985 499 décrit des compositions comprenant des composés de type diazamérocyanine. Il est indiqué que ces composés peuvent être associés à des colorants nitrobenzéniques.

Or, la demanderesse vient maintenant de découvrir qu'il est possible d'obtenir de nouvelles teintures capables de conduire à des colorations puissantes, peu sélectives et résistant bien aux diverses agressions que peuvent subir les cheveux, en associant au moins un colorant direct cationique convenablement sélectionné, et au moins un colorant direct nitré benzénique.

Cette découverte est à la base de la présente invention.

L'invention a donc pour premier objet une composition prête à l'emploi, pour la teinture des fibres kératiniques et en particulier des fibres kératiniques humaines telles que les cheveux, caractérisée par le fait qu'elle comprend, dans un milieu approprié pour la teinture :
- au moins un colorant direct cationique choisi parmi :
   a) les composés de formule (I) suivante : 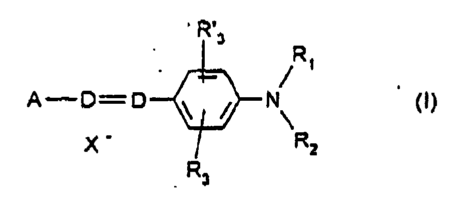 dans laquelle :
      D représente un atome d'azote
      R₁ et R₂, identiques ou différents, représentent un atome d'hydrogène ; un radical alkyle en C₁-C₄ pouvant être substitué par un radical -CN, -OH ou -NH₂ ; ou forment avec un atome de carbone du cycle benzénique un hétérocycle éventuellement oxygéné ou azoté pouvant être substitué par un ou plusieurs radicaux alkyle en C₁-C₄ ; un radical 4'-aminophényle,
      R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
      X⁻ représente un anion de préférence choisi parmi le chlorure, le méthyl sulfate et l'acétate,
      A représente un groupement choisi par les structures A1, A4, A7 et A19 suivantes : 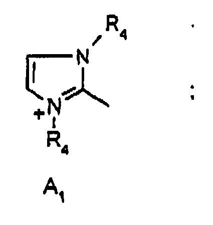 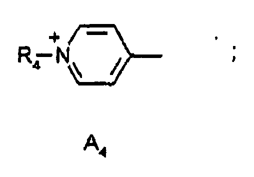 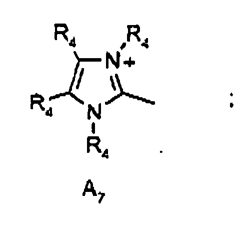 et 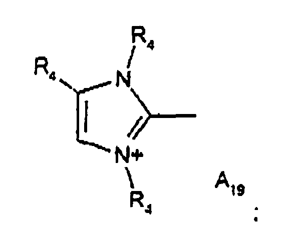 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ et que R₃ est différent d'un radical alcoxy, alors R₁ et R₂ ne désignent pas simultanément un atome d'hydrogène ;
- et au moins un colorant direct nitré benzénique.

La composition tinctoriale prête à l'emploi conforme à l'invention conduit à des colorations puissantes; chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture des fibres kératiniques mettant en oeuvre cette composition tinctoriale prête à l'emploi.

Les colorants directs cationiques de formules (I), utilisables dans les compositions tinctoriales prêtes à l'emploi conformes à l'invention, sont des composés connus et sont décrits par exemple dans les demandes de brevets WO 95/01772, WO 95/15144 et EP-A-0 714 954.

Parmi les colorants directs cationiques de formule (I) utilisables dans les compositions tinctoriales prêtes à l'emploi conformes à l'invention, on peut plus particulièrement citer les composés répondant aux structures (I1) à (I52) suivantes : 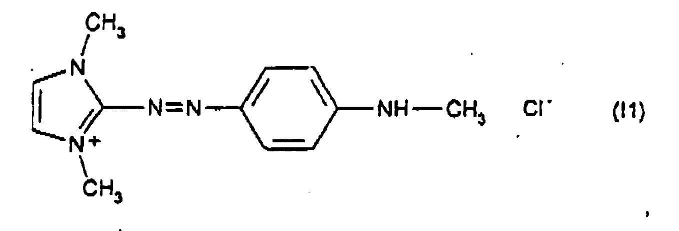 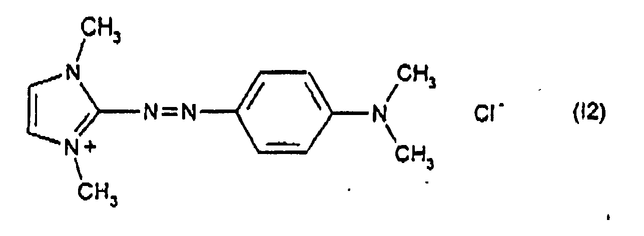 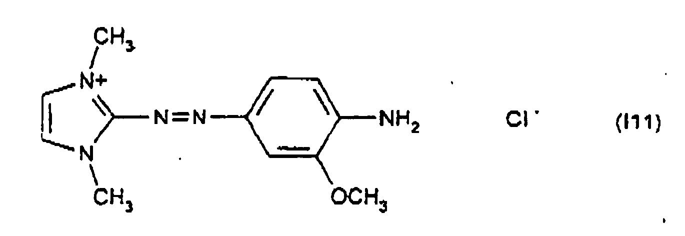 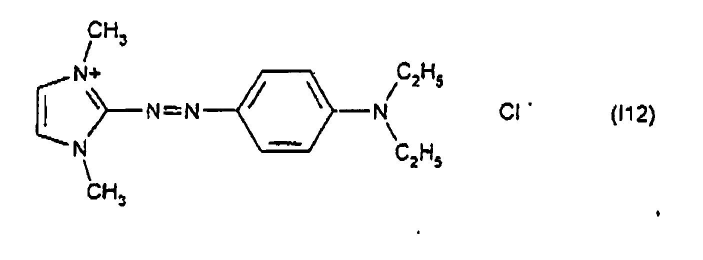 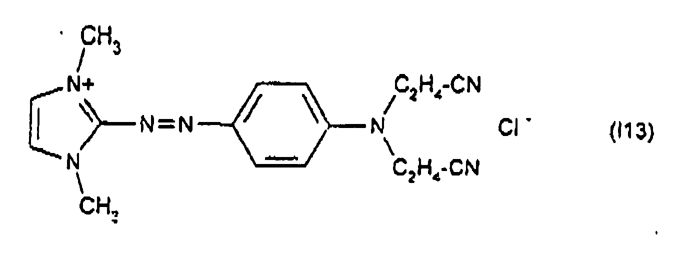 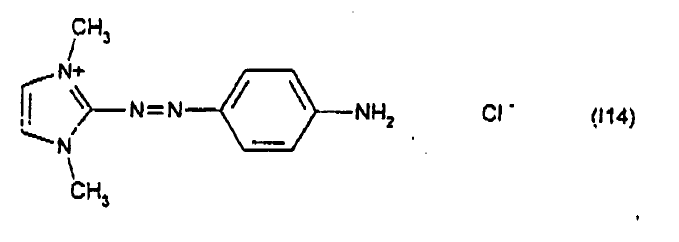 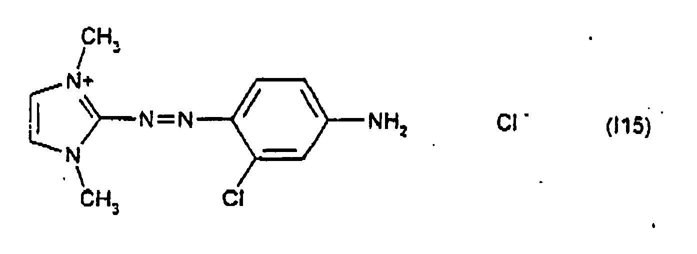 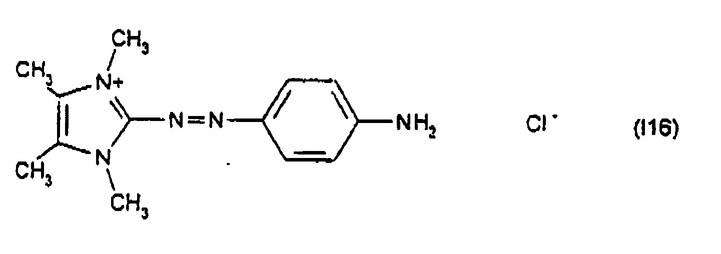 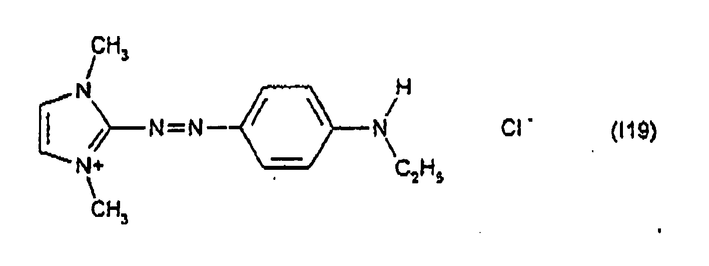 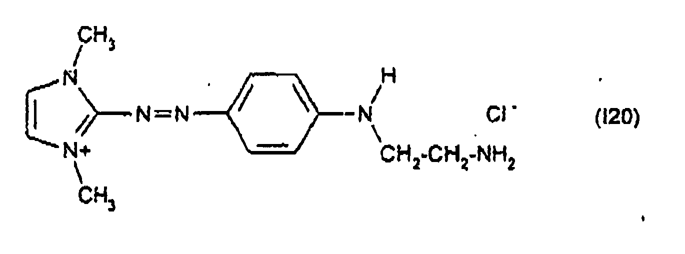 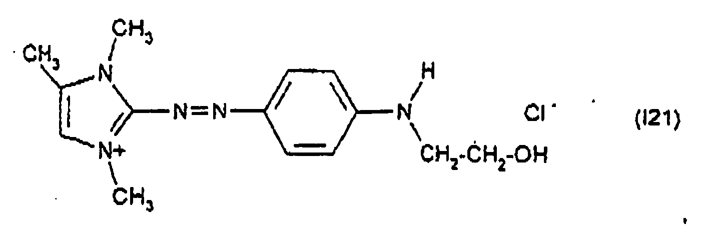 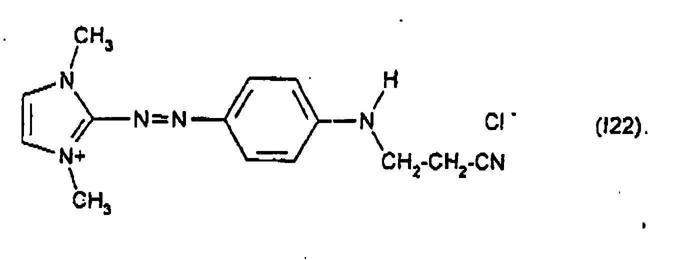 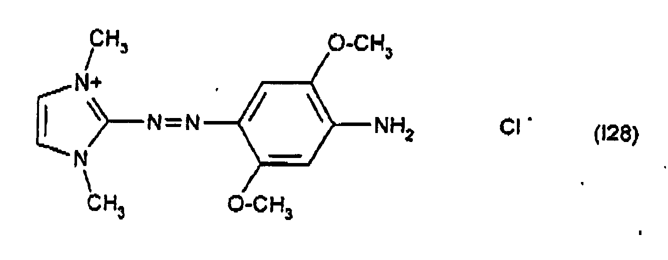 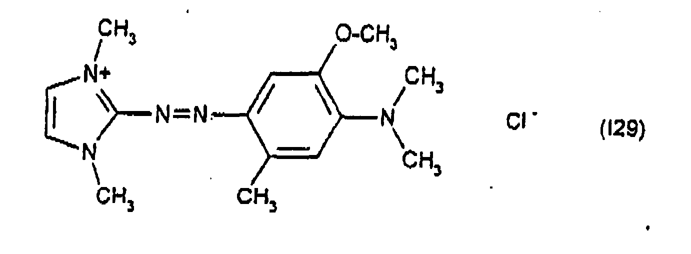 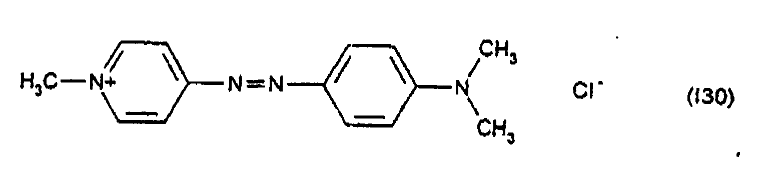 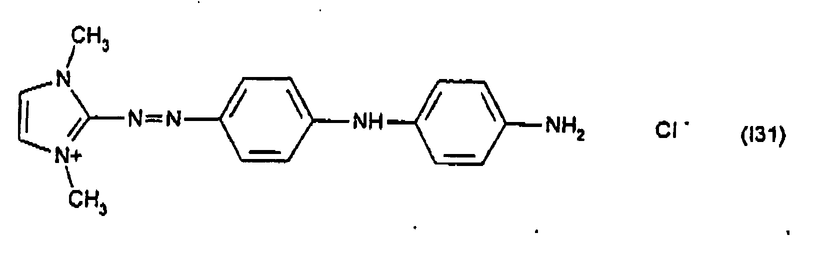 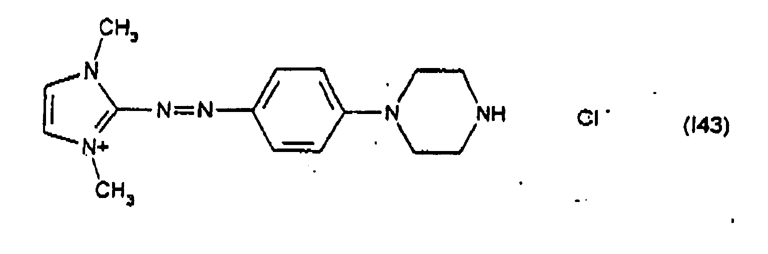 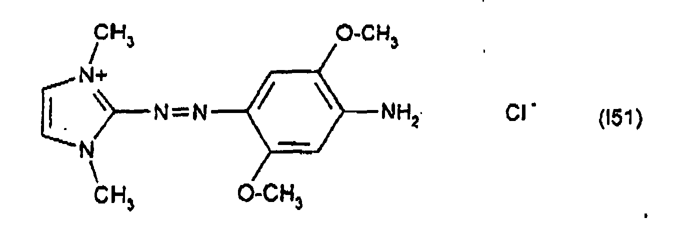 et

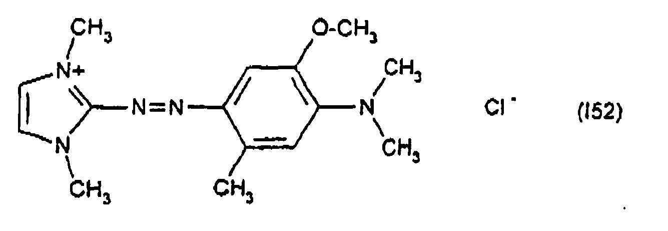

Parmi les composés de structures (I1) à (I52) décrits ci-dessus, on préfère tout particulièrement les composés répondant aux structures (I1), (I2), (I14) et (I31).

Le ou les colorants directs cationiques utilisés selon l'invention, représentent de préférence de 0,001 à 10 % en poids environ du poids total de la composition tinctoriale prête à l'emploi et encore plus préférentiellement de 0,05 à 5 % en poids environ de ce poids.

Le ou les colorants directs nitrés benzéniques pouvant être utilisés dans la composition tinctoriale prête à l'emploi conforme à l'invention sont de préférence choisis parmi les composés de formule (IV) suivante : 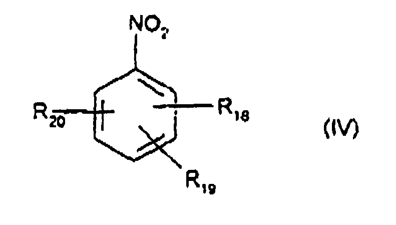 dans laquelle :
- R₁₈ représente un radical amino ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino,
- R₁₉ représente un atome d'hydrogène ; un radical amino ; hydroxyle ; alkyle en C₁-C₄ ; alcoxy en C₁-C₄ ; monohydroxyalkyle en C₁-C₄ ; polyhydroxyalkyle en C₂-C₄ ; monohydroxyalcoxy en C₁-C₄ ; polyhydroxyalcoxy en C₂-C₄ ; aminoalcoxy en C₁-C₄ ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl (C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino ;
- R₂₀ représente un atome d'hydrogène ou d'halogène, un radical alkyle en C₁-C₄, ou un groupement nitro.

Parmi les colorants nitrés benzéniques de formule (IV) ci-dessus, on peut tout particulièrement citer :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- le 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyéthyl)amino 5-méthoxy nitrobenzène,
- le 2-amino 5-β-hydroxyéthyloxy nitrobenzène,
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β-γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-β-γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthyl)amino nitrobenzène,
- le 2-amino 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino-5-amino nitrobenzène,
- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-(β-aminoéthyl)amino 4-hydroxy nitrobenzène,
- l'[hydroxy-2 N-(β-hydroxyéthyl)amino-3 nitro-6] benzyloxy]-2 éthylamine, et
- l'[hydroxy-2 N-(β-hydroxypropyl)amino-3 nitro-6] benzyloxy]-2 éthylamine.

Parmi les colorants nitrés benzéniques de formule (IV) ci-dessus, on préfère tout particulièrement :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N=(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

Le ou les colorants nitrés benzéniques représentent de préférence de 0,0005 à 15 % en poids environ du poids total de la composition tinctoriale prête à l'emploi conforme à l'invention, et encore plus préférentiellement de 0,005 à 10 % en poids environ de ce poids.

La composition tinctoriale prête à l'emploi conforme à l'invention peut en outre renfermer une ou plusieurs bases d'oxydation et/ou un ou plusieurs coupleurs. Ces bases d'oxydation peuvent notamment être choisies parmi les paraphénylènediamines, les para-aminophénols, les orthophénylènediamines et les bases hétérocycliques telles que par exemple les dérivés pyridiniques, les dérivés pyrimidiniques, les dérivés pyrazoliques, et les dérivés pyrazolopyrimidiniques. Les coupleurs peuvent notamment être choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Lorsqu'elles sont présentes, la ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids.

Lorsqu'ils sont présents, le ou les coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates, les tartrates, les lactates et les acétates.

Lorsqu'une ou plusieurs bases d'oxydation et/ou un ou plusieurs coupleurs sont utilisés, alors la composition tinctoriale prête à l'emploi peut en outre renfermer au moins un agent oxydant choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux électrons.

Parmi les oxydo-réductases à 2 électrons pouvant être utilisées à titre d'agent oxydant dans la composition tinctoriale prête à l'emploi conforme à l'invention, on peut plus particulièrement citer les pyranose oxydases, les glucose oxydases, les glycérol oxydases, les lactates oxydases, les pyruvate oxydases, et les uricases.

Selon l'invention, l'utilisation des uricases d'origine animale, microbiologique ou biotechnologique est particulièrement préférée.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

Lorsqu'elles sont utilisées, la ou les oxydo-réductases à 2 électrons représentent de préférence de 0,01 à 20 % en poids environ du poids total de la composition tinctoriale prête à l'emploi, et encore plus préférentiellement de 0,1 à 5 % en poids environ de ce poids.

Lorsqu'une enzyme de type oxydo-réductase à 2 électrons est utilisée conformément à l'invention, la composition tinctoriale prête à l'emploi peut en outre renfermer un ou plusieurs donneurs pour ladite enzyme.

Selon l'invention, on entend par donneur, les différents substrats participant au fonctionnement de ladite ou desdites oxydo-réductases à 2 électrons.

La nature du donneur (ou substrat) utilisé varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Lorsqu'ils sont utilisés, le ou les donneurs (ou substrats) représentent de préférence de 0,01 à 20 % en poids environ du poids total de la composition tinctoriale prête à l'emploi conforme à l'invention et encore plus préférentiellement de 0,1 à 5 % en environ de ce poids.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique ou le phénoxyéthanol, les produits analogues et leurs mélanges.

Le pH de la composition prête à l'emploi conforme à l'invention est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanofamines telles que les mono-, di- et triéthanolamines, le 2-méthyl-2-amino-1-propanol ainsi que leurs dérivés, les hydroxydes de sodium ou de potassium et les composés de formule (V) suivante : 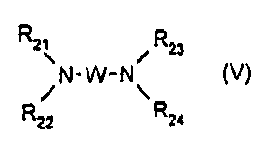 dans laquelle W est un reste propylène éventuellement substitué par un groupement hydroxyle ou un radical alkyle en C₁-C₄ ; R₂₁, R₂₂, R₂₃ et R₂₄, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que par exemple des agents antioxydants, des agents de pénétration, des agents séquestrants, des parfums, des tampons, des agents dispersants, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

Lorsque la composition tinctoriale prête à l'emploi conforme à l'invention renferme au moins une base d'oxydation et/ou au moins un coupleur et au moins un agent oxydant, elle doit alors être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, et lorsque la composition tinctoriale conforme à l'invention renferme au moins une base d'oxydation et/ou au moins un coupleur, le procédé comporte une étape préliminaire consistant à stocker sous forme séparée, d'une part, une composition (A) comprenant, dans un milieu approprié pour la teinture, au moins un colorant direct cationique tel que défini précédemment, au moins un colorant direct nitré benzénique et au moins une base d'oxydation et/ou au moins un coupleur et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 et 2 DE TEINTURE

On a préparé les compositions tinctoriales prêtes à l'emploi suivantes (teneurs en grammes) :

| **COMPOSITION** | **1** | **2** |
|---|---|---|
| 2-amino 5-hydroxy nitrobenzène (colorant direct nitré benzénique) | 0,35 | - |
| 2-N-(β-hydroxyéthyl)amino 5-amino nitro benzène (colorant direct nitré benzénique) | - | 0,25 |
| Colorant direct cationique orangé de structure (I4) | 0,065 | - |
| Colorant direct cationique rouge de structure (I1) | - | 0,04 |
| Support de teinture commun (*) | (*) | (*) |
| Eau déminéralisée q.s.p. | 100 g | 100 g |

| | | |
|---|---|---|
| (*) : Support de teinture commun : - Ethanol 20,0 g - Nonyl phénol oxyéthyléné par 9 moles d'oxyde d'éthylène vendu sous la dénomination IGEPAL NR 9 OR par la société RHODIA CHEMIE 8,0 g - 2-amino-2-méthyl-1-propanol q.s. pH = 7,5 | | |

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans les nuances figurant dans le tableau ci-après :

| EXEMPLE | Nuance obtenue |
|---|---|
| 1 | Cuivré |
| 2 | Acajou rouge |

## Revendications

1. Composition prête à l'emploi, pour la teinture des fibres kératiniques et en particulier des fibres kératiniques humaines telles que les cheveux, **caractérisée par le fait qu'**elle comprend, dans un milieu approprié pour la teinture :
- au moins un colorant direct cationique choisi parmi :
a) les composés de formule (I) suivante : 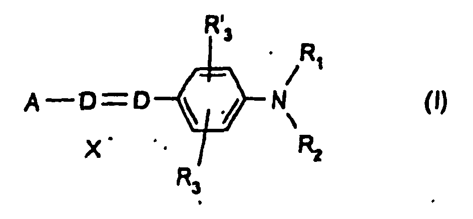 dans laquelle:
D représente un atome d'azote,
R₁ et R₂, identiques ou différents, représentent un atome d'hydrogène ; un radical alkyle en C₁-C₄ pouvant être substitué par un radical -CN, -OH ou -NH₂ ; ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
X⁻ représente un anion de préférence choisi parmi le chlorure, le méthyl sulfate et l'acétate,
A représente un groupement choisi par les structures A1, A4, A7 et A19 suivantes : 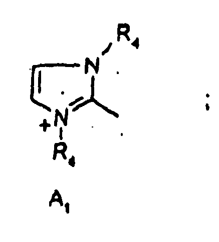 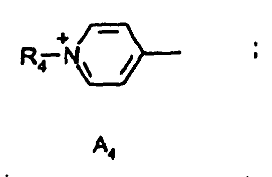 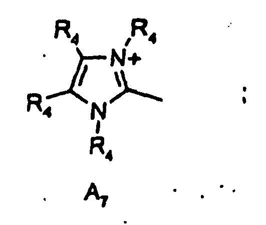 et 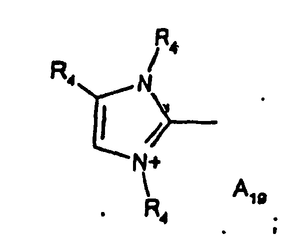 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ et que R₃ est différent d'un radical alcoxy, alors R₁ et R₂ ne désignent pas simultanément un atome d'hydrogène ;
- et au moins un colorant direct nitré benzénique.

2. Composition selon la revendication 1, **caractérisée par le fait que** les colorants directs cationiques de formule (I) sont choisis parmi les composés répondant aux structures (I1) à (I52) suivantes : 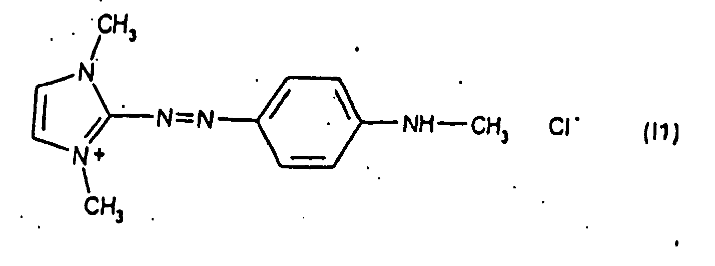 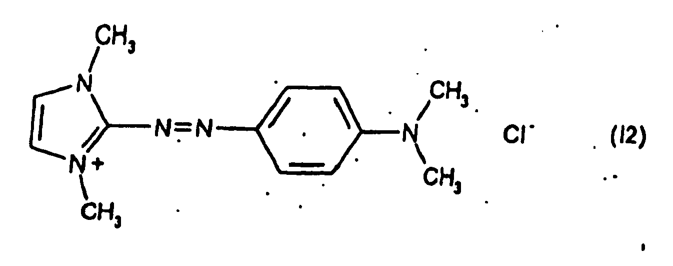 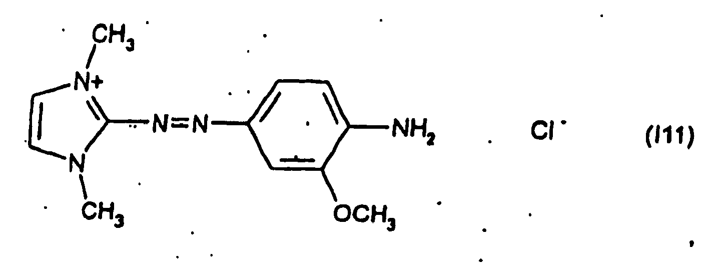 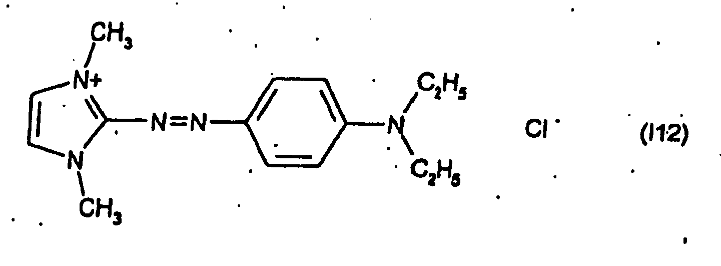 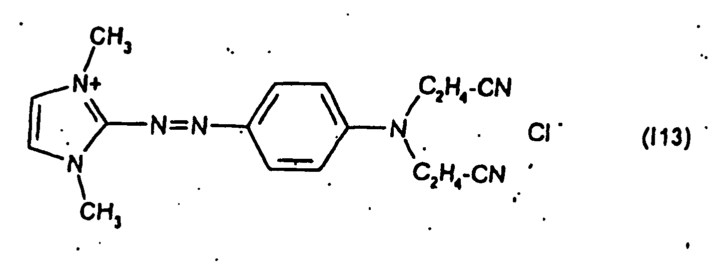 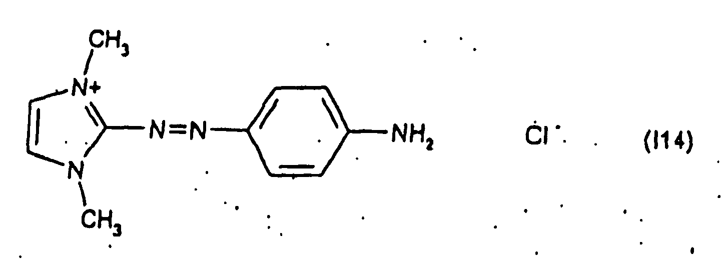 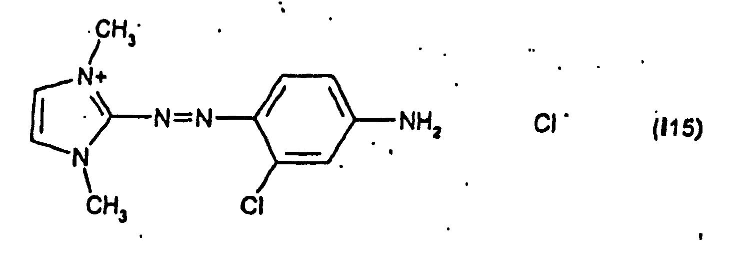 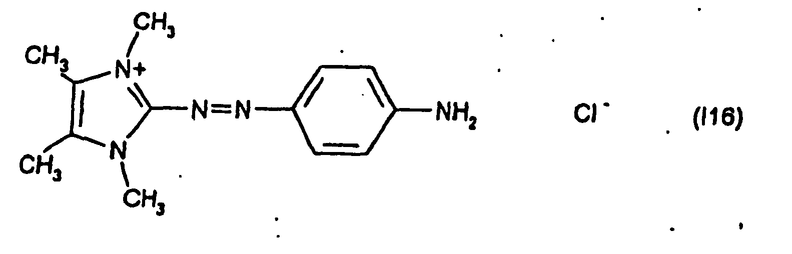 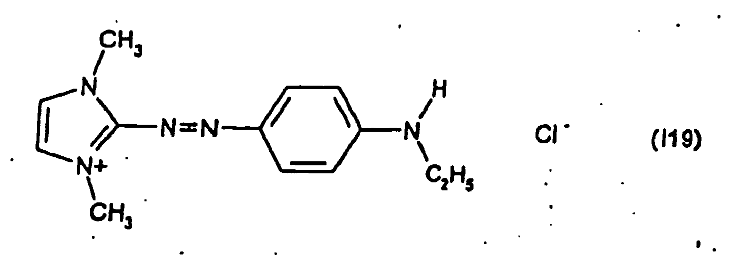 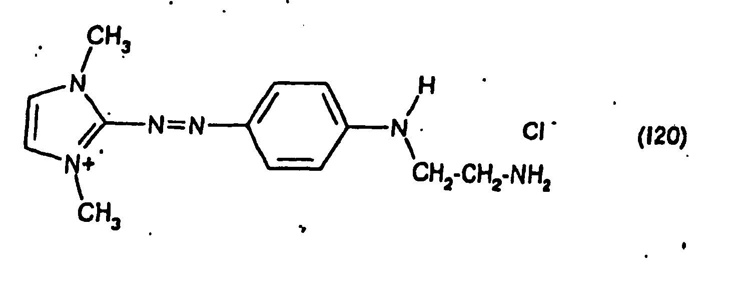 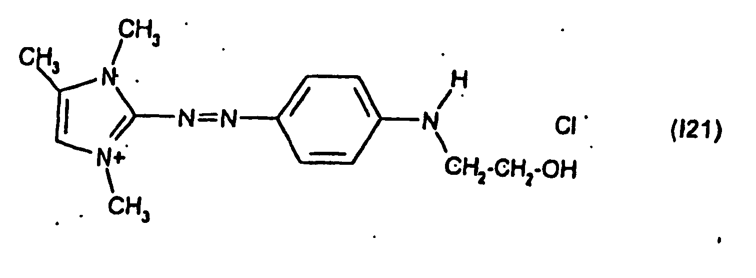 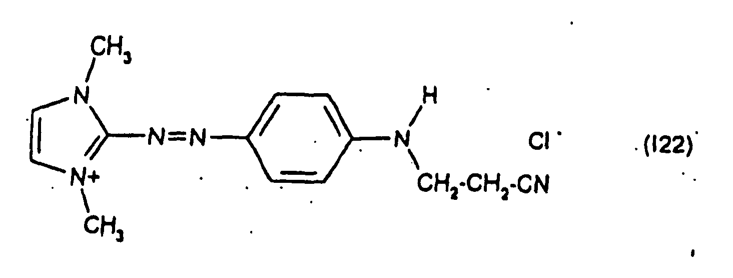 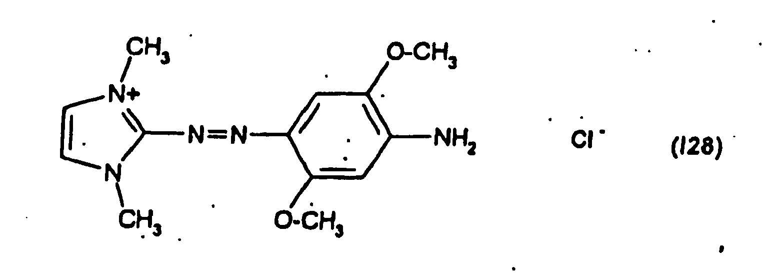 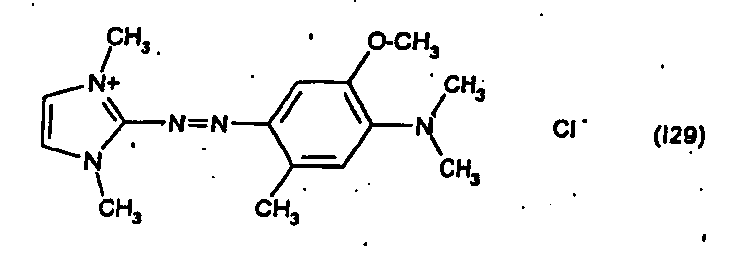 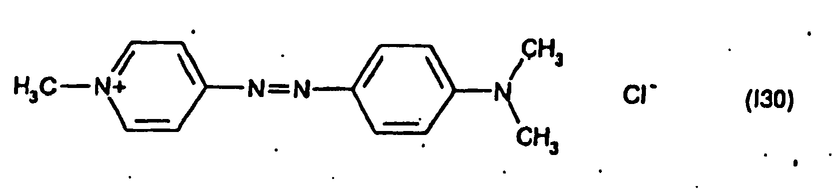 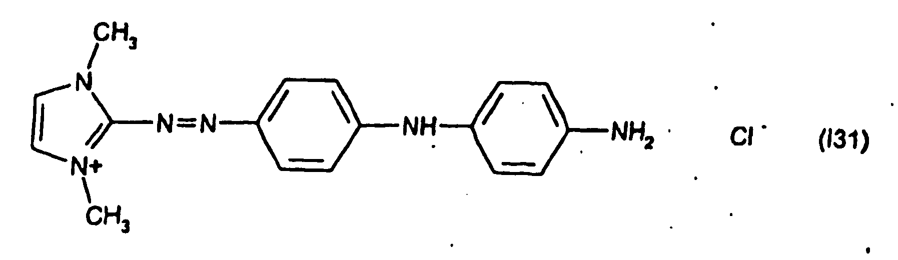 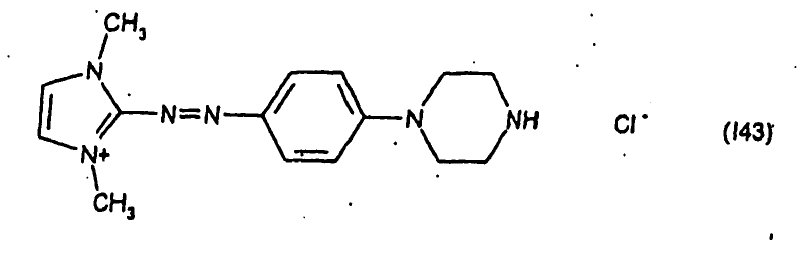 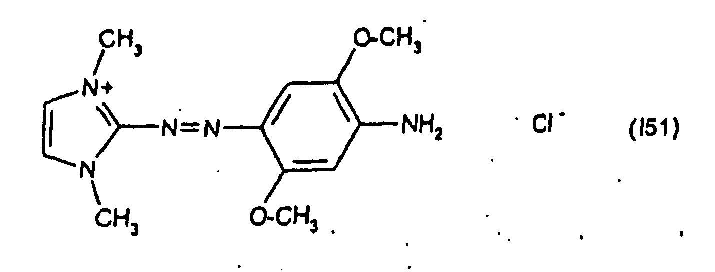 et

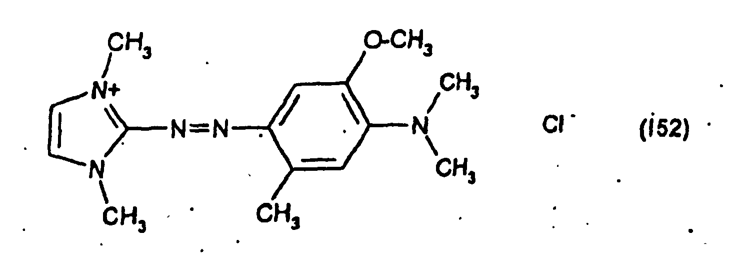

3. Composition selon l'une quelconque des revendications précédentes, **caractérisée par le fait que** le ou les colorants directs cationiques représentent de 0,001 à 10 % en poids du poids total de la composition tinctoriale prête à l'emploi.

4. Composition selon la revendication 3, **caractérisée par le fait que** le ou les colorants directs cationiques représentent de 0,05 à 5 % en poids du poids total de la composition tinctoriale prête à l'emploi.

5. Composition selon l'une quelconque des revendications précédentes, **caractérisée par le fait que** le ou les colorants directs nitrés benzéniques sont choisis parmi les composés de formule (IV) suivante : 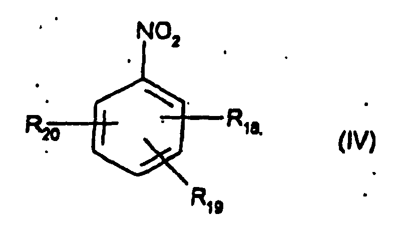 dans laquelle:
- R₁₈ représente un radiçal amino ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino,
- R₁₉ représente un atome d'hydrogène ; un radical amine ; hydroxyle ; alkyle en C₁-C₄ ; alcoxy en C₁-C₄ ; monohydroxyalkyle. en C₁-C₄ ; polyhydroxyalkyle en C₂-C₄ ; monohydroxyalcoxy en C₁-C₄ ; polyhydroxyalcoxy en C₂-C₄ ; aminoalcoxy en C₁-C₄ ; un radical amino monosubstitué ou disubstitué par un radical alkyle en C₁-C₄, monohydroxyalkyle en C₁-C₄, polyhydroxyalkyle en C₂-C₄, aminoalkyle en C₁-C₄, monoalkyl(C₁-C₄)amino alkyle en C₁-C₄, dialkyl(C₁-C₄)amino alkyle en C₁-C₄, uréidoalkyle en C₁-C₄, aryle, aryle dont le cycle aryle est substitué par un ou plusieurs radicaux hydroxyle, carboxyle, amino ou dialkyl(C₁-C₄)amino ;
- R₂₀ représente un atome d'hydrogène ou d'halogène, un radical, alkyle en C₁-C₄, ou un groupement nitro.

6. Composition selon la revendication 5, **caractérisée par le fait que** les colorants nitrés benzéniques de formule (IV) sont choisis parmi :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- 2,4-diamino nitrobenzène,
- le 3,4-diamino nitrobenzène,
- le 2,5-diamino nitrobenzène,
- le 3-amino 4-hydroxy nitrobenzène,
- le 4-amino 3-hydroxy nitrobenzène,
- le 5-amino 2-hydroxy nitrobenzène,
- le 2-amino 5-hydroxy nitrobenzène,
- le 4-amino 3-hydroxy nitrobenzène,
- le 5-amino 2-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2,5-N,N'-(β-hydroxyéthyl)amino nitrobenzène,
- le 2-N-(β-hydroxyéthyl)amino 5-N,N-bis-(β-hydroxyéthyl)amino nitrobenzène,
- le 2-amino 5-N-(méthyl)amino nitrobenzène,
- le 2-N-(méthyl)amino 5-N,N-bis-(β-hydroxyéthyl)amino nitrobenzène,
- le 2-N-(méthyl)amino 5-(N-méthyl N-β-hydroxyéthyl)amino nitrobenzène,
- lé 2,5-N,N'-(β-hydroxyéthyl)amino nitrobenzène,
- le 2-N-(β-hydroxyéthyl)amino 5-hydroxy nitrobenzène,
- le 3-méthoxy 4-N-(β-hydroxyéthyl)amino nitrobenzène,
- le 2-N-(méthyl)amino 4-β-hydroxyéthyloxy nitrobenzène,
- le 2-amino 3-méthyl nitrobenzène,
- le 2-N-(β-hydroxyéthyl)amino 5-amino nitrobenzène,
- le 2-amino 4-chloro 5-N-(β-hydroxyéthyl)amino nitrobenzène,
- le 2-amino 4-méthyl 5-N-(β-hydroxyéthyl)amino nitrobenzène,
- le 2-amino 4-méthyl 5-N-(méthyl)amino nitrobenzène,
- le 2-N-(β-hydroxyèthyl)amino 5-méthoxy nitrobenzène,
- le 2-amino 5-β-hydroxyéthyloxy nitrobenzène,
- le 2-N-(β-hydroxyéthyl)amino nitrobenzène,
- le 3-amino 4-N-(β-hydroxyéthyl)amino nitrobenzène,
- le 3-β-hydroxyéthyloxy 4-N-(β-hydroxyéthyl)amino nitrobenzène,
- le 2-N-(méthyl)amino 4-β,γ-dihydroxypropyloxy nitrobenzène,
- le 2-N-(β-hydroxyéthyl)amino 5-β-hydroxyéthyloxy nitrobenzène,
- le 2-N-(β-hydroxyéthyl)amino 5-(β,γ-dihydroxypropyloxy nitrobenzène,
- le 2-hydroxy 4-N-(β-hydroxyéthyl)amino nitrobenzène,
- le 2-N-(méthyl)amino 4-méthyl 5-amino nitrobenzène,
- le 2-amino 4-isopropyl 5-N-(méthyl)amino nitrobenzène,
- le 2-N-(méthyl)amino 5-(N-méthyl N-β,γ-dihydroxypropyl)amino nitrobenzène,
- le 3-N-(β-hydroxyéthyl)amino 4-N-(β-hydroxyéthyl)amino nitrobenzène,
- le 2-amino 4-méthyl 5-N-(β,γ-dihydroxypropyl)amino nitrobenzène,
- le 2-amino 4-méthyl 5-hydroxy nitrobenzène,
- le 2-N-(β-hydroxyéthyl)amino 4-N-(β-hydroxyéthyl)amino nitrobenzène,
- le 2-amino 5-N-(β-aminoéthyl)amino nitrobenzène,
- le 2-N-(β-aminoéthyl)amino 5-méthoxy nitrobenzène,
- le 2-N-(méthyl)amino 5-N-(β-aminoéthyl)amino nitrobenzène,
- le 2-N-(β-aminoéthyl)amino 4-N,N-(diméthyl)amino nitrobenzène,
- le 3-amino 4-N-(β-aminoéthyl)amino nitrobenzène,
- le 2-amino 4-méthyl 5-N-(β-aminoéthyl)amino nitrobenzène,
- le 2-N-(β-aminoéthyl)amino 5-N,N-bis-(β-hydroxyéthyl)amino nitrobenzène,
- le 3-β-aminoéthyloxy 4-amino nitrobenzène,
- le 2-N-(méthyl)amino 5-(N-δ-amino n-butyl)amino nitrobenzène,
- le 2-N-(γ-amino n-propyl)amino 5-N,N-(diméthyl)amino nitrobenzène,
- le 3-méthoxy 4-N-(β-aminoéthyl)amino nitrobenzène,
- le 2-N-(β-aminoéthyl)amino 5-amino nitrobenzène,
- le 2-amino 4-chloro 5-N-(β-aminoéthyl)amino nitrobenzène,
- le 2-N-(β-aminoéthyl)amino 4-méthoxy nitrobenzène,
- le 2-N-(β-aminoéthyl)amino nitrobenzène,
- le 2-N-(β-aminoéthyl)amino 5-N-(β-aminoéthyl)amino nitrobenzène,
- le 2-N-(β-aminoéthyl)amino 4-β-hydroxyéthyloxy nitrobenzène,
- le 3-β-hydroxyéthyloxy 4-N-(β-aminoéthyl)amino nitrobenzène,
- le 2-amino 5-aminoéthyloxy nitrobenzène,
- le 3-hydroxy 4-N-(β-aminoéthyl)amino nitrobenzène,
- le 2-N-(β-aminoéthyl)amino 5-β-hydroxyéthyloxy nitrobenzène,
- le 2-N-β-aminoéthyl)amino 4-hydroxy nitrobenzène,
- l'[hydroxy-2 N-β-hydroxyéthyl)amino-3 nitro-6] benzyloxy]-2 éthylamine, et
- l'[hydroxy-2 N-β-hydroxypropyl)amino-3 nitro-6] benzyloxy]-2 éthylamine.

7. Composition selon la revendication 6, **caractérisée par le fait que** les colorants nitrés benzéniques de formule (IV) sont choisis parmi :
- le 2-amino 4-méthyl 5-N-(β-hydroxyéthyl)amino nitrobenzène,
- le 4-N-(β-uréidoéthyl)amino nitrobenzène,
- le 4-(N-éthyl N-β-hydroxyéthyl)amino 1-N-(β-hydroxyéthyl)amino nitrobenzène,
- le 2-N-(β-hydroxyéthyl)amino 5-méthyl nitrobenzène,
- le 5-chloro 3-N-(éthyl)amino 4-hydroxy nitrobenzène,
- le 5-amino 3-chloro 4-hydroxy nitrobenzène,
- le 2-N-(γ-hydroxypropyl)amino 5-N,N-bis-(β-hydroxyéthyl)amino nitrobenzène,
- le 5-hydroxy 2-N-(γ-hydroxypropyl)amino nitrobenzène,
- le 1,3-bis-(β-hydroxyéthyl)amino 4-chloro 6-nitro benzène,
- le 3,4-diamino nitrobenzène,
- le 2-amino 5-hydroxy nitrobenzène,
- le 2-amino 3-hydroxy nitrobenzène,
- le 2-amino 5-N-(β-hydroxyéthyl)amino nitrobenzène,
- le 2-amino 5-N,N-bis-(β-hydroxyéthyl)amino nitrobenzène,
- le 2-N-(β-hydroxyéthyl)amino 5-N,N-bis-(β-hydroxyéthyl)amino nitrobenzène,
- le 2-N-(β-hydroxyéthyl)amino 5-hydroxy nitrobenzène,
- le 2-N-(β-hydroxyéthyl)amino 5-amino nitrobenzène,
- le 2-N-(β-aminoéthyl)amino 4-méthoxy nitrobenzène, et
- le 2-N-(β-aminoéthyl)amino 5-β-hydroxyéthyloxy nitrobenzène.

8. Composition selon l'une quelconque des revendications précédentes, **caractérisée par le fait que** le ou les colorants nitrés benzéniques représentent de 0,0005 à 15 % en poids de la composition tinctoriale prête à l'emploi.

9. Composition selon la revendication 8, **caractérisée par le fait que** le ou les colorants nitrés benzéniques représentent de 0,005 à 10 % en poids de la composition tinctoriale prête à l'emploi.

10. Composition selon l'une quelconque des revendications précédentes, **caractérisée par le fait qu'**elle renferme une ou plusieurs bases d'oxydation choisies parmi les paraphénylènediamines, les para-aminophénols, les orthophénylènediamines et les bases hétérocycliques et/ou un ou plusieurs coupleurs choisis parmi les méta-phénylènediamines, les méta-aminophénols, les métadiphénois, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

11. Composition selon la revendication 10, **caractérisée par le fait que** la ou les bases d'oxydation représentent de 0,0005 à 12 % en poids du poids total de la composition tinctoriale prête à l'emploi et que le ou les coupleurs représentent de 0,0001 à 10 % en poids du poids total de la composition tinctoriale prête à l'emploi.

12. Composition selon la revendication 11, **caractérisée par le fait que** la ou les bases d'oxydation représentent de 0,005 à 8 % en poids du poids total de la composition tinctoriale prête à l'emploi et que le ou les coupleurs représentent de 0,005 à 5 % en poids du poids total de la composition tinctoriale prête à l'emploi.

13. Composition selon l'une quelconque des revendications 10 à 12, **caractérisée par le fait que** les sels d'addition avec un acide sont choisis parmi les chlorhydrates, les bromhydrates, les sulfates, les tartrates, les lactates et les acétates.

14. Composition selon l'une quelconque des revendications 10 à 13, **caractérisée par le fait qu'**elle renferme au moins un agent oxydant.

15. Composition selon la revendication 14, **caractérisée par le fait que** l'agent oxydant est choisi parmi le peroxyde d'hydrogène, le peroxyde d'urée, les bromates de métaux alcalins, les persels tels que les perborates et persulfates, et les enzymes.

16. Composition selon la revendication 15, **caractérisée par le fait que** les enzymes sont choisies parmi les peroxydases et les oxydo-réductases à deux électrons.

17. Composition selon la revendication 16, **caractérisée par le fait que** les oxydo-réductases à deux électrons sont choisies parmi les pyranose oxydases, les glucose oxydases, les glycérol oxydases, les lactates oxydases, les pyruvate oxydases, et les uricases.

18. Composition selon la revendication 16 ou 17, **caractérisée par le fait que** l'oxydo-réductases à 2 électrons est choisie parmi les uricases d'origine animale, microbiologique ou biotechnologique.

19. Composition selon l'une quelconque des revendications 16 à 18, **caractérisée par le fait que** la ou les oxydo-réductases à 2 électrons représentent de 0,01 à 20 % en poids du poids total de la composition tinctoriale prête à l'emploi.

20. Composition selon la revendication 19, **caractérisée par le fait que** la ou les oxydo-réductases à 2 électrons représentent de 0,1 à 5 % en poids du poids total de la composition tinctoriale prête à l'emploi.

21. Composition selon l'une quelconque des revendications 18 à 20, **caractérisée par le fait qu'**elle renferme un donneur (ou substrat) pour ladite oxydo-réductase à 2 électrons, choisi parmi l'acide urique et ses sels.

22. Composition selon l'une quelconque des revendications précédentes, **caractérisée par le fait que** le milieu approprié pour la teinture est constitué par de l'eau ou par un mélange d'eau et d'au moins un solvant organique.

23. Composition selon l'une quelconque des revendications précédentes, **caractérisée par le fait qu'**elle présente un pH compris 5 et 11.

24. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

25. Procédé selon la revendication 24, **caractérisé par le fait qu'**il comporte une étape préliminaire consistant à stocker sous forme séparée, d'une part, une composition (A) comprenant, dans un milieu approprié pour la teinture, au moins un colorant direct cationique tel que défini à l'une quelconque des revendications 1 à 4, au moins un colorant direct nitré benzénique et au moins une base d'oxydation et/ou au moins un coupleur et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

26. Dispositif à plusieurs compartiments ou "kit" de teinture, **caractérisé par le fait qu'**il comporte un premier compartiment renfermant la composition (A) telle que définie dans la revendication 25 et un second compartiment renfermant la composition (B) telle que définie dans la revendication 25.

## Claims

1. Ready-to-use composition for dyeing keratin fibres, and in particular human keratin fibres such as the hair, **characterised in that** it comprises, in a medium which is suitable for dyeing:
- at least one cationic direct dye chosen from the compounds of formula (I) below: 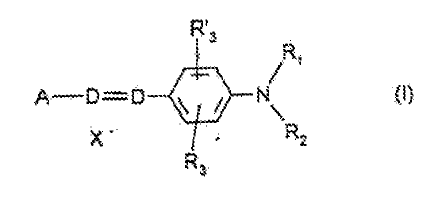 in which:
D represents a nitrogen atom,
R¹ and R₂, which may be identical or different, represent a hydrogen atom; a C₁-C₄ alkyl radical which can be substituted with a -CN, -OH or -NH₂ radical; or form, with a carbon atom of the benzene ring, an optionally oxygenated or nitrogenous heterocycle which can be substituted with one or more C₁-C₄ alkyl radicals; a 4'-aminophenyl radical,
R₃ and R'₃, which may be identical or different, represent a hydrogen or halogen atom chosen from chlorine, bromine, iodine and fluorine, or a cyano, C₁-C₄ alkoxy or acetyloxy radical,
X⁻ represents an anion preferably chosen from chloride, methyl sulfate and acetate,
A represents a group chosen from structures A₁, A₄, A₇ and A₁₉ below: 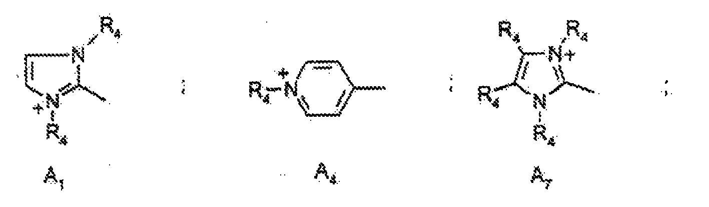
and 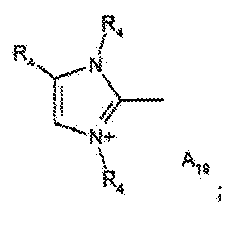 in which R₄ represents a C₁-C₄ alkyl radical which can be substituted with a hydroxyl radical and R₅ represents a C₁-C₄ alkoxy radical, with the proviso that when D represents -CH, when A represents A₄ and when R₃ is other than an alkoxy radical, then R₁ and R₂ do not simultaneously denote a hydrogen atom;
- and at least one nitrobenzene direct dye.

2. Composition according to Claim 1, **characterized in that** the cationic direct dyes of formula (I) are chosen from the compounds corresponding to structures (I1) and (I2), (I11) to (I16), (I19) to (I22), (I38) to (I31), (I43), (I51) and (I52) below: 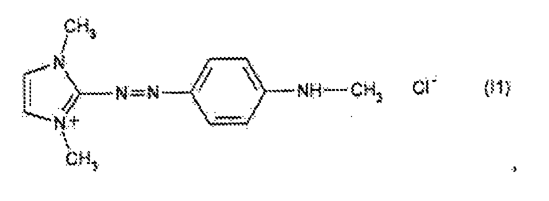 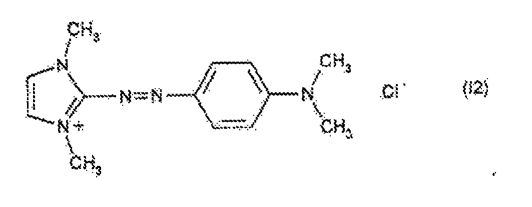 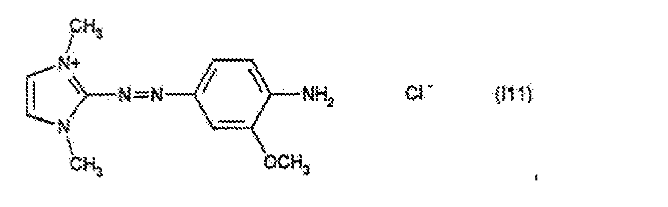 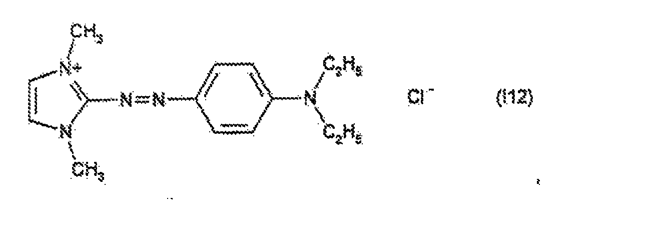 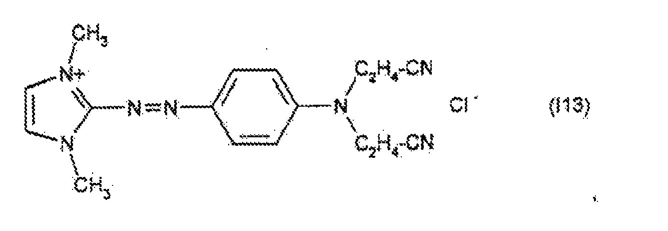 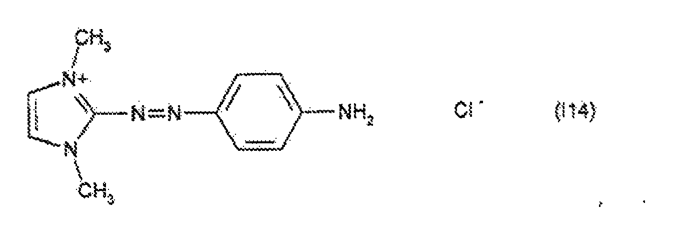 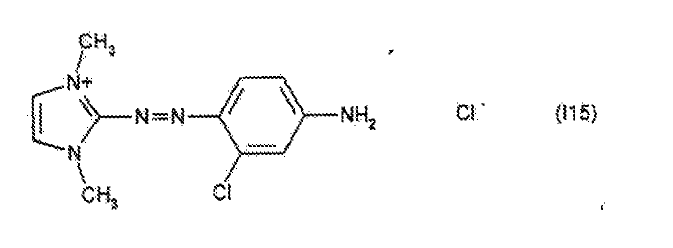 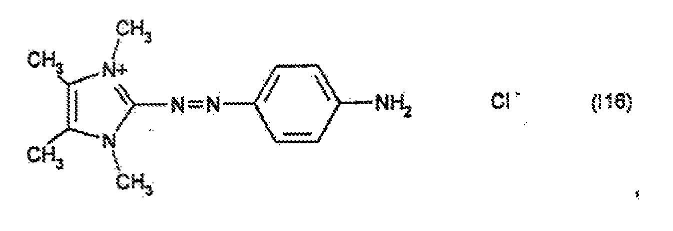 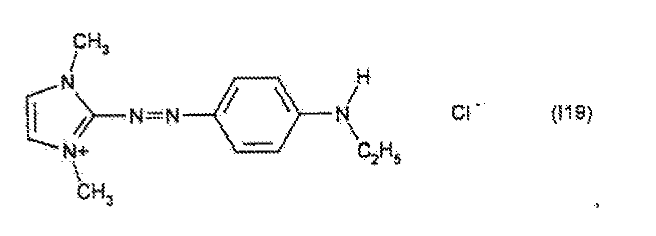 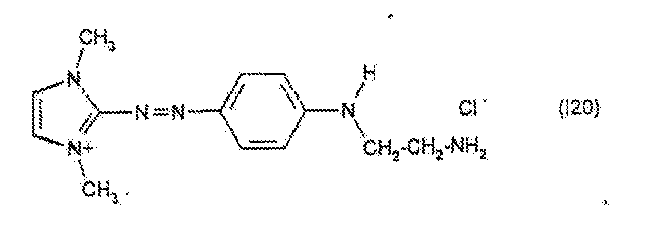 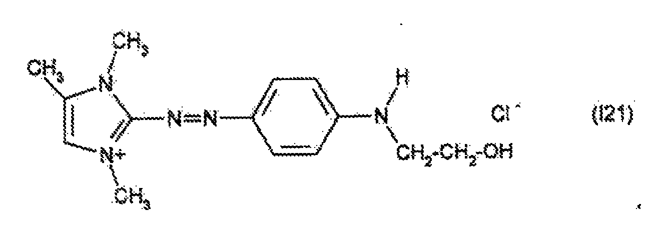 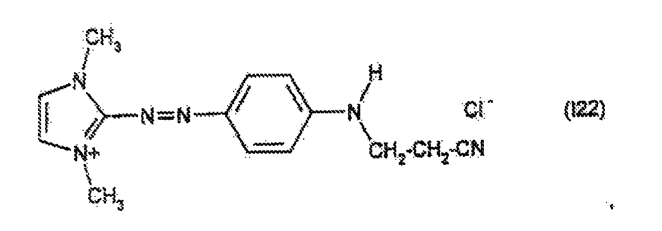 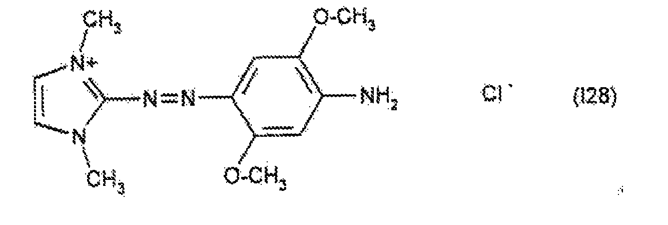 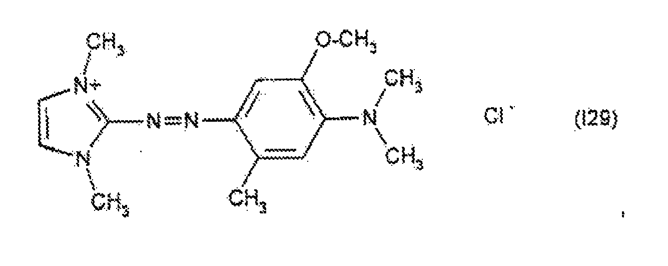 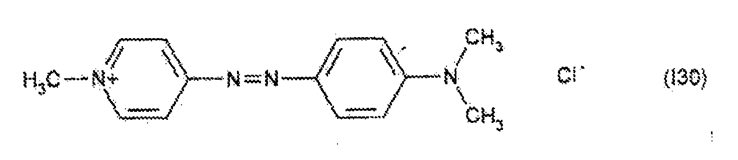 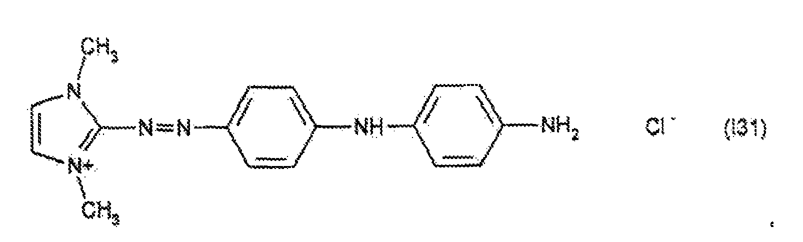 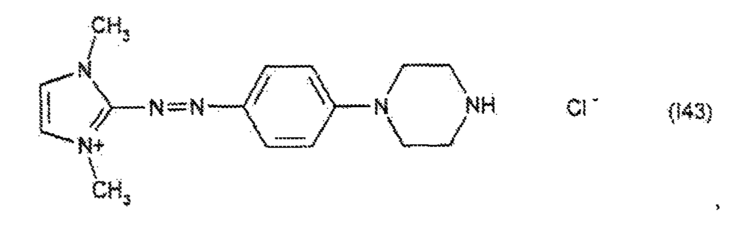 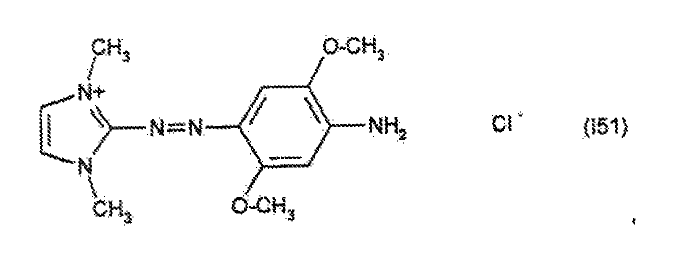 and

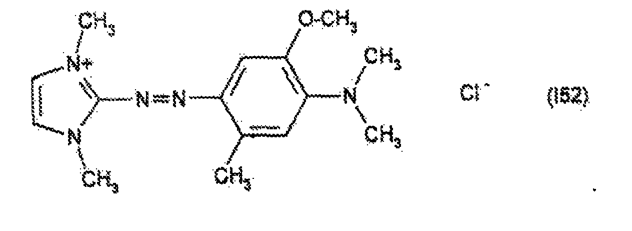

3. Composition according to either of the preceding claims, **characterized in that** the cationic direct dye(s) represent(s) from 0.001 to 10% by weight relative to the total weight of the ready-to-use dye composition.

4. Composition according to Claim 3, **characterized in that** the cationic direct dye(s) represent(s) from 0.05 to 5% by weight relative to the total weight of the ready-to-use dye composition.

5. Composition according to any one of the preceding claims, **characterized in that** the nitrobenzene direct dye(s) is (are) chosen from the compounds of formula (IV) below: 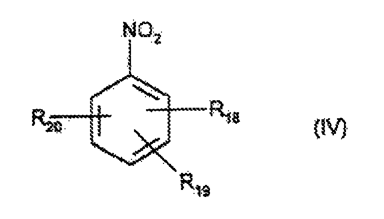 in which:
- R₁₈ represents an amino radical; an amino radical monosubstituted or disubstituted with a C₁-C₄ alkyl, C₁-C₄ monohydroxyalkyl, C₂-C₄ polyhydroxyalkyl, C₁-C₄ aminoalkyl, mono (C₁-C₄) alkylamino(C₁-C₄)alkyl, di (C₁-C₄) alkylamino(C₁-C₄)alkyl or ureido(C₁-C₄)alkyl or aryl radical or an aryl radical in which the aryl ring is substituted with one or more hydroxyl, carboxyl, amino or di (C₁-C₄) alkylamino radicals,
- R₁₉ represents a hydrogen atom; an amino radical; hydroxyl radical; C₁-C₄ alkyl radical; C₁-C₄ alkoxy radical; C₁-C₄ monohydroxyalkyl radical; C₂-C₄ polyhydroxyalkyl radical; C₁-C₄ monohydroxyalkoxy radical; C₂-C₄ polyhydroxyalkoxy radical; C₁-C₄ aminoalkoxy radical; an amino radical monosubstituted or disubstituted with a C₁-C₄ alkyl, C₁-C₄ monohydroxyalkyl, C₂-C₄ polyhydroxyalkyl, C₁-C₄ aminoalkyl, mono(C₁-C₄)alkylamino(C₁-C₄)alkyl, di (C₁-C₄)alkylamino(C₁-C₄) alkyl or ureido(C₁-C₄) alkyl or aryl radical or an aryl radical in which the aryl ring is substituted with one or more hydroxy, carboxyl, amino or di(C₁-C₄)alkylamino radicals;
- R₂₀ represents a hydrogen or halogen atom, a C₁-C₄ alkyl radical or a nitro group.

6. Composition according to Claim 5, **characterized in that** the nitrobenzene dyes of formula (IV) are chosen from:
- 2-amino-4-methyl-5-N-(β-hydroxyethyl)aminonitrobenzene,
- 4-N-(β-ureidoethyl)aminonitrobenzene,
- 4-(N-ethyl-N-β-hydroxyethyl)amino-1-N-(β-hydyoxyethyl) aminonitrobenzene,
- 2-N-(β-hydroxyethyl)amino-5-methylnitrobenzene,
- 5-chloro-3-N-(ethyl)amino-4-hydroxynitrobenzene,
- 5-amino-3-chloro-4-hydroxynitrobenzene,
- 2-N-(γ-hydroxypropyl)amino-5-N,N-bis(β-hydroxyethyl)-aminonitrobenzene,
- 5-hydroxy-2-N-(γ-hydroxypropyl) aminonitrobenzene,
- 1,3-bis(β-hydroxyethy)amino-4-chloro-6-nitrobenzene,
- 2,4-diaminonitrobenzene,
- 3,4-diaminonitrobenzene,
- 2,5-diaminonitrobenzene,
- 3-amino-4-hydroxynitrobenzene,
- 4-amino-3-hydroxynitrobenzene,
- 5-amino-2-hydroxynitrobenzene,
- 2-amino-5-hydroxynitrobenzene,
- 4-amino-3-hydroxynitrobenzene,
- 5-amino-2-hydroxynitrobenzene,
- 2-amino-3-hydroxynitrobenzene,
- 2-amino-5-N-(β-hydroxyethyl)aminonitrobenzene,
- 2-amino-5-N,N-bis(β*-*hydroxyethyl)aminonitrobenzene,
- 2,5-N,N'-(β-hydroxyethyl)aminonitrobenzene,
- 2-N-(β-hydroxyethyl)amino-5-N,N-bis(β-hydroxyethyl)-aminonitrobenzene,
- 2-amino-5-N-(methyl)aminonitrobenzene,
- 2-N-(methyl)amino-5-N,N-bis(β-hydroxyethyl)aminonitrobenzene,
- 2-N-(methyl)amino-5-(N-methyl-N-β-hydroxyethyl)-aminonitrobenzene,
- 2,5-N,N'-(β-hydroxyethyl)aminonitrobenzene,
- 2-N-(β-hydroxyethyl)amino-5-hydroxynitrobenzene,
- 3-methoxy-4-N-(β-hydroxyethyl)aminonitrobenzene,
- 2-N-(methyl)amino-4-β-hydroxyethylnitrobenzene,
- 2-amino-3-methylnitrobenzene,
- 2-N-(β-hydroxyethyl)amino-5-aminonitrobenzene,
- 2-amino-4-chloro-5-N-(β-hydroxyethyl)aminonitrobenzene,
- 2-amino-4-methyl-5-N-(β-hydroxyethyl)aminonitrobenzene,
- 2-amino-4-methyl-5-N-(methyl)aminonitrobenzene,
- 2-N-(β-hydroxyethyl)amino-5-methoxynitrobenzene.
- 2-amino-5-β-hydroxyethyloxynitrobenzene,
- 2-N-(β-hydroxyethyl)aminonitrobenzene,
- 3-amino-4-N-(β-hydroxyethyl)aminonitrobenzen,
- 3-β-hydroxyethyloxy-4-N-(β-hydroxyethyl)aminonitrobenzene,
- 2-N-(methyl)amino-4-β-γ-dihydroxypropyloxynitrobenzene,
- 2-N-(β-hydroxyethyl)amino-5-β-hydroxyethyloxynitrobenzene,
- 2-N-(β-hydroxyethyl)amino-5-β,γ-dihydroxypropyloxynitrobenzene,
- 2-hydroxy-4-N-(β-hydroxyethyl)aminonitrobenzene,
- 2-N-(methyl)amino-4-methyl-5-aminonitrobenzene,
- 2-amino-4-isopropyl-5-N-(methyl)-aminonitrobenzene,
- 2-N-(methyl)amino-5-(N-methyl-N-β,γ-dihydroxypropyl)-aminonitrobenzene,
- 3-N-(β-hydroxyethyl)amino-4-N-(β-hydroxyethyl)-amino-nitrobenzene,
- 2-amino-4-methyl-5-N-(β,γ-dihydroxypropyl)aminonitrobenzene,
- 3-amino-4-methyl-5-hydroxynitrobenzene,
- 2-N-(β-hydroxyethyl)amino-4-N-(β-hydroxyethyl)aminonitrobenzene,
- 2-amino-5-N-(β-aminoethyl)aminonitrobenzene,
- 2-N-(β-aminoethyl)amino-5-methoxynitrobenzene,
- 2-N-(methyl)amino-5-N-(β-aminoethyl)aminonitrobenzene,
- 2-N-(β-aminoethyl)amino-4-N,N*-(*dimethyl)aminonitro, benzene,
- 3-amino-4-N-(β-aminoethyl)aminonitrobenzene,
- 2-amino-4-methyl-5-N-(β-aminoethyl)aminonitrobenzene,
- 2-N-(β-aminoethyl)amino-5-N,N-bis(β-hydroxyethyl)-aminonitrobenzene,
- 3-β-aminoethyloxy-4-aminonitrobenzene,
- 2-N-(methyl)-amino-5-(N-δ-amino-n-butyl)-aminonitrobenzene,
- 2-N-(γ-amino-n-propyl)amino-5-N,N-(dimethyl)aminonitrobenzene,
- 3-methoxy-4-N-(β-aminoethyl)aminonitrobenzene,
- 2-N-(β-aminoethyl)amino-5-aminonitrobenzene,
- 2-amino-4-chloro-5-N-(β-aminoethyl)aminonitrobenzene,
- 2-N-(β-aminoethyl)amino-4-methoxynitrobenzene,
- 2-N-(β-aminoethyl)aminonitrobenzene,
- 2-N-(β-aminoethyl)amino-5-N-(β-aminoethyl)aminonitrobenzene,
- 2-N-(β-aminoethyl)amino-4-β-hydroxyethyloxynitrobenzene,
- 3-β-hydroxyethyloxy-4-N-(β-aminoethyl)aminonitrobenzene,
- 2-amino-5-aminoethyloxynitrobenzene,
- 3-hydroxy-4-N-(β-aminoethyl)aminonitrobenzene,
- 2-N-(β-aminoethyl)amino-5-β-hydroxyethyloxynitrobenzene,
- 2-N-(β-aminoethyl)amino-4-hydroxynitrobenzene,
- 2-{[2-hydroxy-3-N-(β-hydroxyethyl)amino-6-nitro]benzyloxy}ethylamine, and
- 2-{[2-hydroxy-3-N-(β-hydroxypropyl)amino-6-nitro]benzyloxy}ethylamine

7. Composition according to Claim 6, **characterized in that** the nitrobenzene dyes of formula (IV) are chosen from:
- 2-amino-4-methyl-5-N-(β-hydroxyethyl)aminonitrobenzene,
- 4-N-(β-ureidoethyl)aminonitrobenzene,
- 4-(N-ethyl-N-β-hydroxyethyl)amino-1-N-(β-hydroxyethyl)aminonitrobenzene,
- 2-N-(β-hydroxyethyl)amino-5-methylnitrobenzene,
- 5-chloro-3-N-(ethyl)amino-4-hydroxynitrobenzene,
- 5-amino-3-chloro-4-hydyoxynitrobenzene,
- 2-N-(γ-hydroxypropyl)amino-5-N,N-bis(β-hydroxyethyl)-aminonitrobenzene,
- 5-hydroxy-2-N-(γ-hydroxypropyl)aminonitrobensene,
- 1,3-bis(β-hydroxyethyl)amino-4-chloro-6-nitrobenzene,
- 3,4-diaminonitrobenzene,
- 2-amino-5-hydroxynitrobenzene,
- 2-amino-3-hydroxynitrobenzene,
- 2-amino-5-N-(β-hydroxyethyl)aminonitrobenzene,
- 2-amino-5-N,N-bis(β-hydroxyethyl)aminonitrobenzene,
- 2-N-(β-hydroxyethyl)amino-5-N,N-bis(β-hydroxyethyl)-aminonitrobenzene,
- 3-N-(β-hydroxyethyl)amino-5-hydroxynitrobenzene,
- 2-N-(β-hydroxyethyl)amino-5-aminonitrobenzene,
- 2-N-(β-aminoethyl)amino-4-methoxynitrobenzene, and
- 2-N-(β-aminoethyl)amino-5-β-hydroxyethyloxynitrobenzene.

8. Composition according to any one of the preceding claims, **characterised in that** the nitrobenzene dye (s) represent (s) from 0.0005 to 15% by weight of the ready-to-use dye composition.

9. Composition according to Claim 8, **characterised in that** the nitrobenzene dye (s) represent (s) from 0.005 to 10% by weight of the ready-to-use dye composition.

10. Composition according to any one of the preceding claims, **characterized in that** it contains one or more oxidation bases chosen from para-phenylenediamines, para-aminophenols, ortho-phenylenediamines and heterocyclic bases and/or one or more couplers chosen from meta-phenylenediamines, meta-aminophenols, meta-diphenols, heterocyclic couplers such as, for example, indole derivatives, indoline derivatives, benzimidazole derivatives, benzomorpholine derivatives, sesamol derivatives, pyridine, pyrimidine and pyrazole derivatives, and the addition salts thereof with an acid.

11. Composition according to Claim 10, **characterised in that** the oxidation base (s) represent (s) from 0.0005 to 12% by weight relative to the total weight of the ready-to-use dye composition and **in that** the coupler (s) represent (s) from 0.0001 to 10% by weight relative to the total weight of the ready-to-use dye composition.

12. Composition according to Claim 11, **characterized in that** the oxidation base (s) represent (s) from 0.005 to 8% by weight relative to the total weight of the ready-to-use dye composition and **in that** the coupler (s) represent (s) from 0.005 to 5% by weight relative to the total weight of the ready-to-use dye composition.

13. Composition according to any one of Claims 10 to 12, **characterised in that** the addition salts with an acid are chosen from the hydrochlorides, hydrobromides, sulfates, tartrates, lactates and acetates.

14. Composition according to any one of Claims 10 to 13, **characterized in that** it contains at least one oxidizing agent.

15. Composition according to Claim 14, **characterized in that** the oxidizing agent is chosen from hydrogen peroxide, urea peroxide, alkali metal bromates, persalts such as perborates and persulfates, and enzymes.

16. Composition according to Claim 15, **characterized in that** the enzymes are chosen from peroxidases and two-electron oxidoreductases.

17. Composition according to Claim 16, **characterised in that** the two-electron oxidoreductases are chosen from pyranose oxidases, 1 glucose oxidases, glycerol oxidases, lactate oxidases, pyruvate oxidases and uricases.

18. Composition according to Claim 16 or 17, **characterised in that** the 2-electron oxidereductase is chosen from uricases of animal, microbiological or biotechnological origin.

19. Composition according to any one of Claims 16 to 18, **characterised in that** the 2-electron oxidoreductase (s) represent (s) from 0.01 to 20% by weight relative to the total weight of the ready-to-use dye composition.

20. Composition according to claim 19, **characterised in that** the 2-electron oxidoreductase (s) represent (s) from 0.1 to 5% by weight relative to the total weight of the ready-to-use dye composition.

21. Composition according to any one of Claims 18 to 20, **characterised in that** it contains a donor (or substrate) for the said 2-electron oxidoreductase, chosen from uric acid and its salts.

22. Composition according to any one of the preceding claims, **characterised in that** the medium which is suitable for dyeing consists of water or a mixture of water and at least one organic solvent.

23. Composition according to any one of the preceding claims, **characterized in that** it has a pH of between 5 and 11.

24. process for dyeing keratin fibres, and in particular human keratin fibres such as the hair, **characterized in that** at least one ready-to-use dye composition as defined in any one of the preceding claims is applied to the said fibres for a period which is sufficient to develop the desired coloration.

25. Process according to Claim 34, **characterised in that** it includes a preliminary step which consists in separately storing, on the one hand, a composition (A) comprising, in a medium which is suitable for dyeing, at least one cationic direct dye as defined, in any one of Claims 1 to 4, at least one nitrobenzene direct dye and at least one oxidation base and/or at least one coupler, and, on the other hand, a composition (B) containing, in a medium which is suitable for dyeing, at least one oxidising agent, and then in mixing them together at the time of use, before applying this mixture to the keratin fibres.

26. Multi-compartment dyeing device or kit, **characterised in that** it includes a first compartment containing composition (A) as defined in Claim 25 and a second compartment containing composition (B) as defined in Claim 25.

## Patentansprüche

1. Gebrauchsfertige Zusammensetzung-zum Färben von Keratinfasern und insbesondere menschlichen Keratinfasern, wie Haaren, **dadurch gekennzeichnet, dass** sie in einem zum Färben geeigneten Medium enthält:
- mindestens einen kationischen Direktfarbstoff, der ausgewählt ist unter:
a) Verbindungen der folgenden Formel (I); 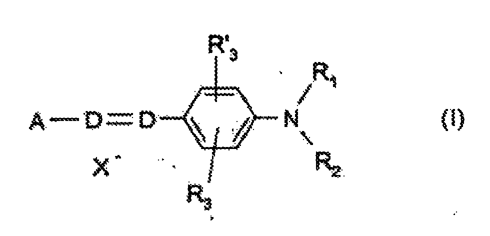 worin bedeuten:
D ein Stickstoffatom,
R₁ und R₂, die gleich oder verschieden sind, ein Wasserstoffatom; eine C₁₋₄-Alkylgruppe, die mit einer Gruppe -CN, -OH oder -NH₂ substituiert sein kann; oder sie bilden mit einem Kohlenstoffatom des Benzolrings einen gegebenenfalls sauerstoff- oder stickstoffhaltigen Heterocyclus, der mit einer oder mehreren C₁₋₄-Alkylgruppen substituiert sein kann; oder eine 4'-Aminophenylgruppe,
R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkoxy oder Acetyloxy,
X ein Anion, das vorzugsweise unter Chlorid, Methylsulfat und Acetat ausgewählt list,
A eine Gruppe, die unter den folgenden Strukturen A₁, A₄, A₇ und A₁₉ ausgewählt ist: 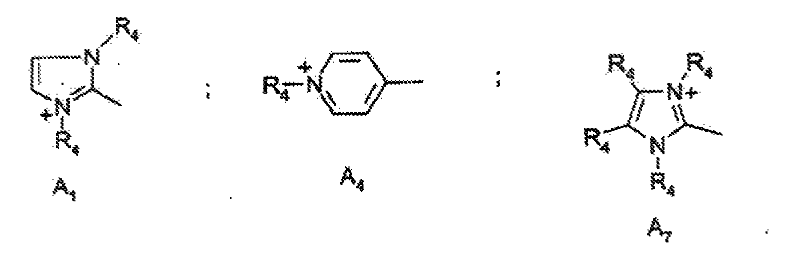
und 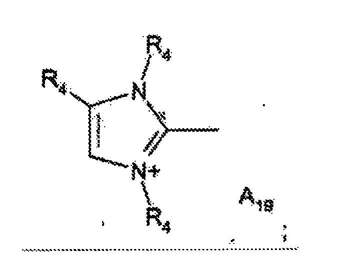 worin die Gruppe R¹ eine C₁₋₄-Alkylgruppe bedeutet, die mit einer Hydroxygruppe substituiert sein kann, und R₅ eine C₁₋₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ bedeutet und R₃ von Alkoxy verschieden ist; und
- mindestens einen Direktfarbstoff Vom nitrierten Benzoltyp.

2. Zusammensetzung nach Anspruch 1, **dadurch gekennzeichnet, dass** die kationischen Direktfarbstoffe der Formel (I) unter den Verbindungen der folgenden Strukturen (11) bis (152) ausgewählt sind:

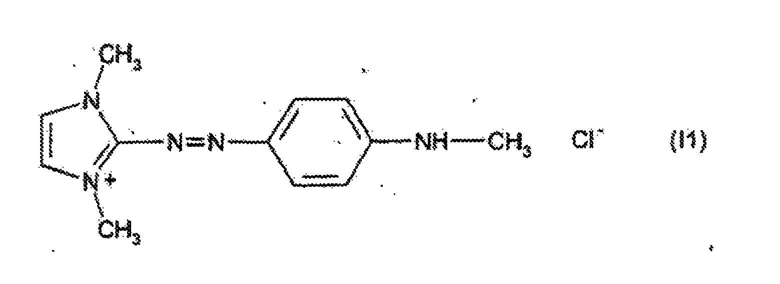

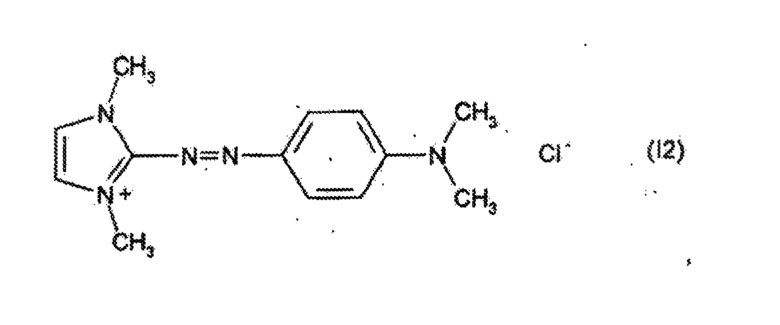

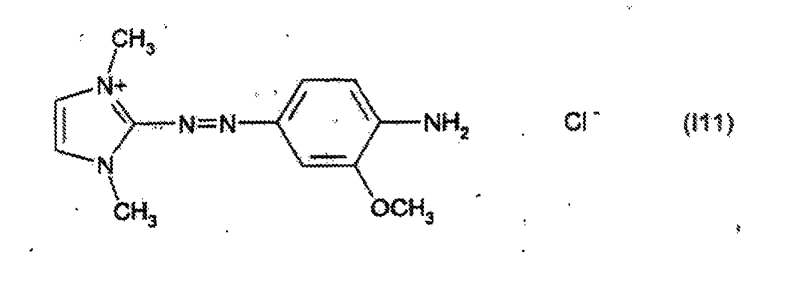

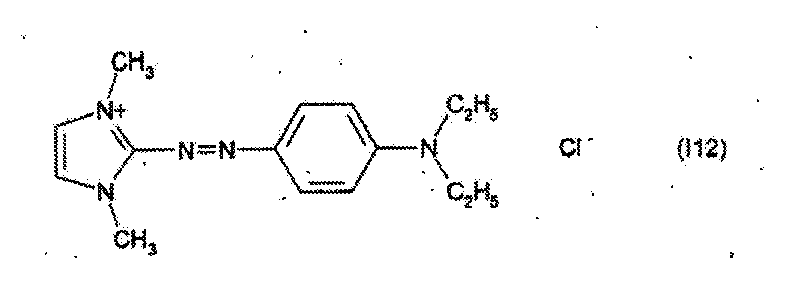

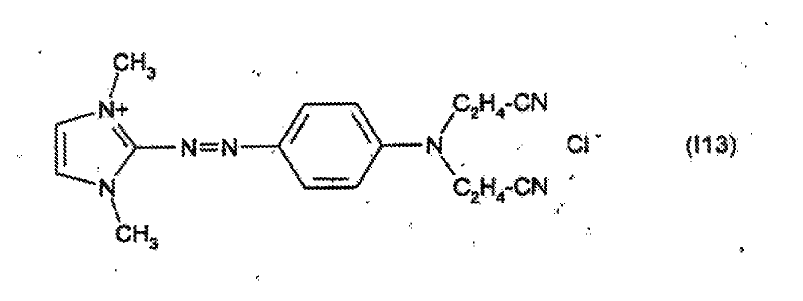

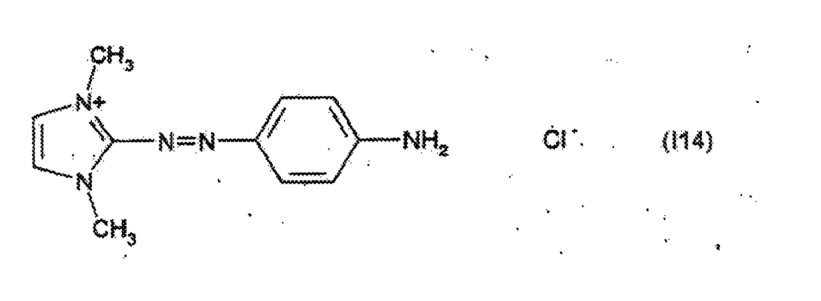

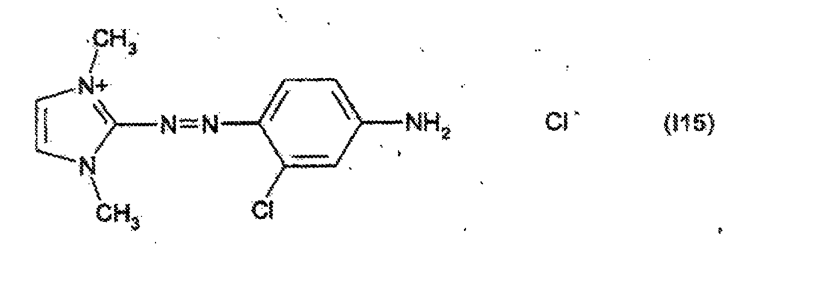

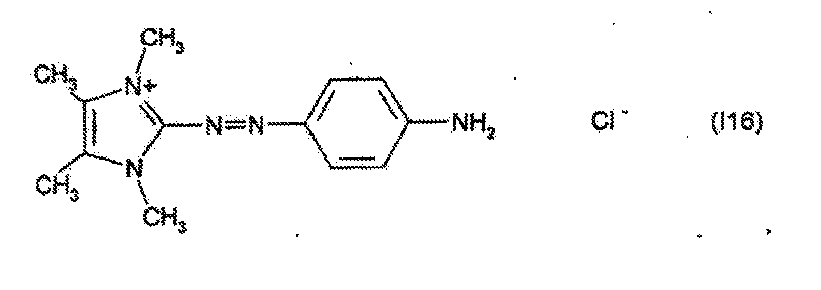

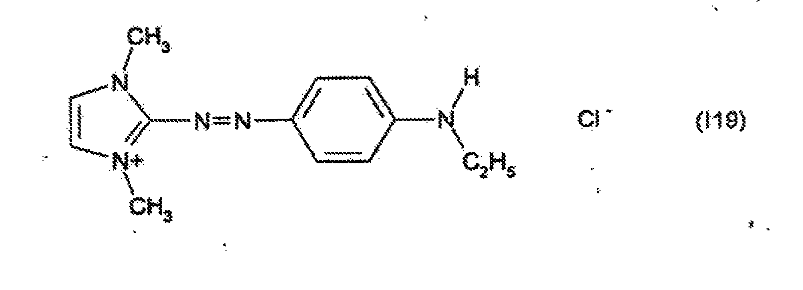

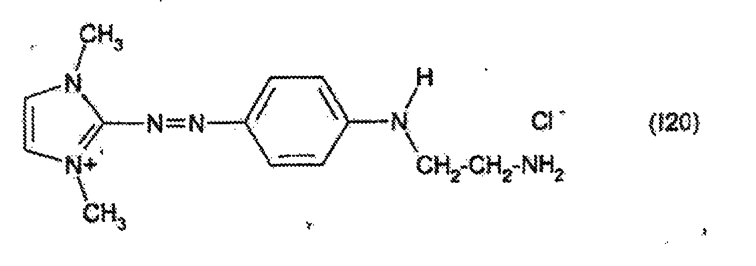

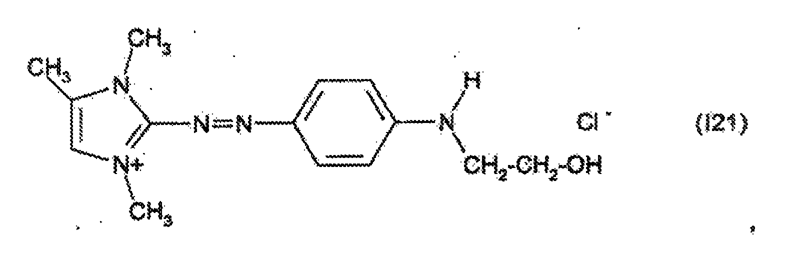

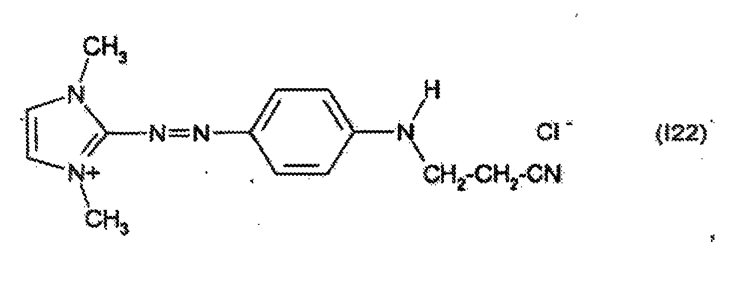

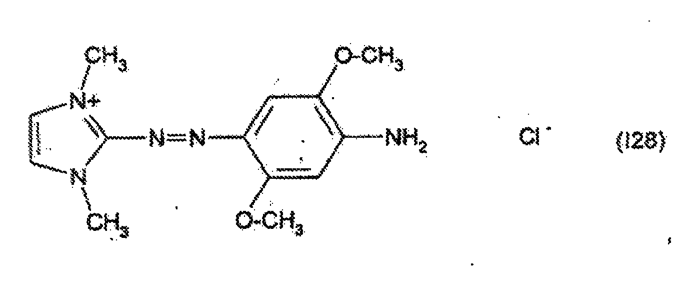

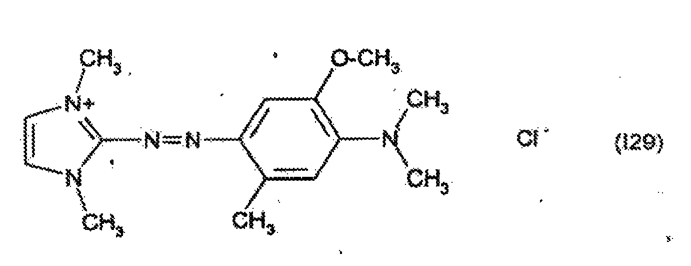

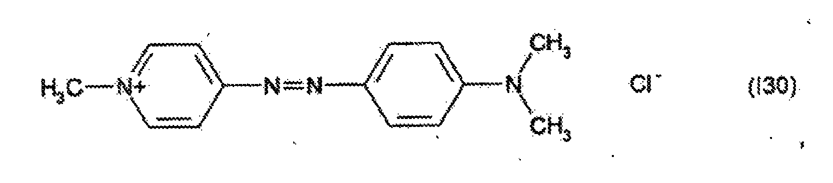

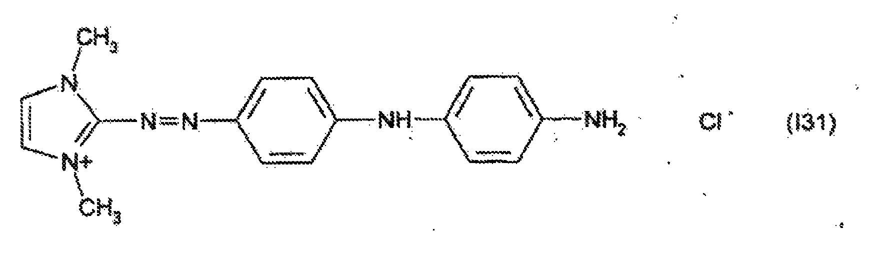

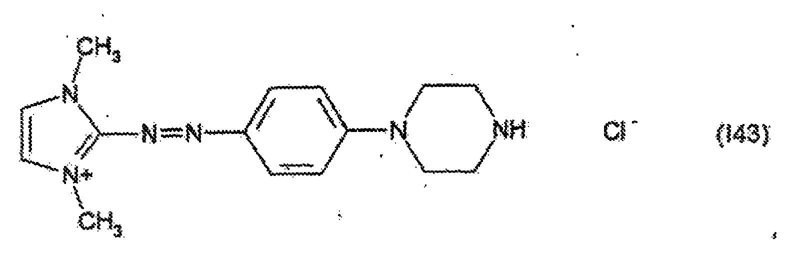

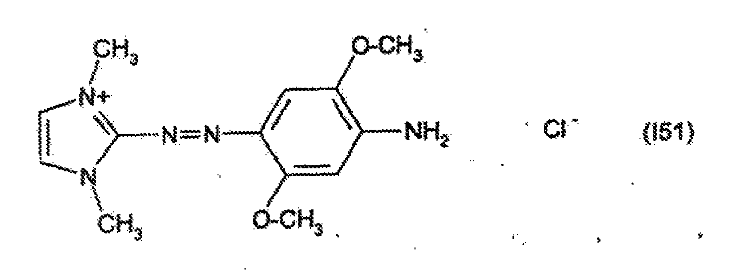

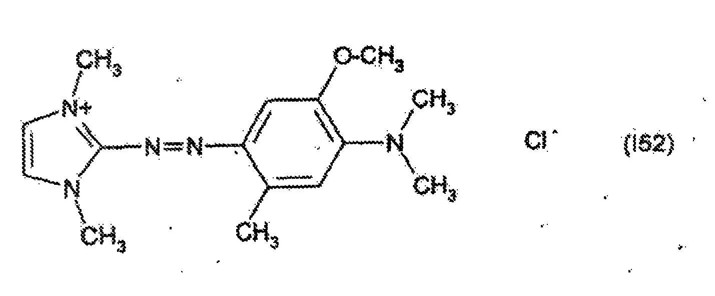

3. Zusammensetzung nach einem der vorhergehenden Ansprüche, **dadurch gekennzeichnet, dass** der oder die kationische(n) Direktfarbstoff(e) 0,001 bis 10 Gew.-% des Gesamtgewichts der gebrauchsfertigen Farbmittelzusammensetzung ausmachen.

4. Zusammensetzung nach Anspruch 3, **dadurch gekennzeichnet, dass** der oder die kationische(n) Direktfarbstoff(e) 0,05 bis 5 Gew.-% des Gesamtgewichts der gebrauchsfertigen Farbmittelzusammensetzung ausmachen.

5. Zusammensetzung nach einem der vorhergehenden Ansprüche, **dadurch gekennzeichnet, dass** der nitriert Benzolfarbstoff oder die nitrierten Benzolfarbstoffe unter den Verbindungen der folgenden Formel (IV) ausgewählt sind: 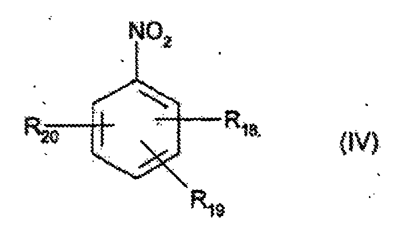 worin bedeuten:
- R₁₈ eine Aminogruppe; eine Aminogruppe, die mono- oder disubstituiert ist mit C₁₋₄-Alkyl, C₁₋₄-Monohydroxyalkyl, C₂₋₄-Polyhydroxyalkyl, C₁₋₄-Aminoalkyl, Mono-C₁₋₄-alkylamino-C₁₋₄ alkyl, Di-C₁₋₄-alkylamino-C₁₋₄-alkyl, C₁₋₄-Ureidoalkyl, Aryl, einer Arylgruppe, deren Arylring mit einer oder mehreren Hydroxygruppen, Carboxygruppen, Aminogruppen oder Di-C₁₋₄-alkylaminogruppen substituiert ist;
- R₁₉ ein Wasserstoffatom; eine Aminogruppe; Hydroxy; C₁₋₄-Alkyl, C₁-₄-Alkoxy, C₁₋₄-Monohydroxyalkyl, C₂₋₄-Polyhydroxyalkyl, C₁₋₄-Monohydroxyalkoxy, C₂₋₄-Polyhydroxyalkoxy, C₁₋₄-Aminoalkoxy, eine Aminogruppe, die mono- oder disubstituiert ist mit C₁₋₄-Alkyl, C₁₋₄-Monohydroxyalkyl, C₂₋₄-Polyhydroxyalkyl, C₁₋₄-Aminoalkyl, Mono-C₁₋₄-alkylamino-C₁₋₄-alkyl Di-C₁₋₄-alkylamino-C₁₋₄-alkyl, C₁₋₄-Ureidoalkyl, Aryl, einer Arylgruppe, deren Arylring mit einer oder mehreren Hydroxygruppen, Carboxygruppen, Aminogruppen oder Di-C₁₋₄-alkylaminogruppen substituiert ist;
- R₂₀ ein Wasserstoffatom; ein Halogenatom; C₁₋₄-Alkyl oder Nitro.

6. Zusammensetzung nach Anspruch 5, **dadurch gekennzeichnet, dass** die nitrierten Benzolfarbstoffe der Formel (IV) unter den folgenden Verbindungen ausgewählt sind:
- 2-Amino-4-methyl-5-N-(β-hydroxyethyl)amino-nitrobenzol,
- 4-N-(β-Ureidoethyl)amino-nitrobenzol
- 4-(N-Ethyl-N-(β-hyrdroxyethyl)amino-1-N-(β-hydroxyethyl)-amino-nitrobenzol,
- 2-N-(β-Hydroxyethyl)amino-5-methyl-nitrobenzol,
- 5-Chlor-3-N-(ethyl)amino-4-hydroxy-nitrobenzol,
- 5-Amino-3-chlor-4-hydroxy-nitrobenzol,
- 2-N-(γ-Hydroxypropyl)amino-5-N,N-bis(β-hydroxyethyl)amino-nitrobenzol,
- 5-Hydroxy-2-N-(γ-hydroxypropyl)amino-nitrobenzol,
- 1,3-Bis(β-hydroxyethyl)amino-4-chlor-6-nitrobenzol,
- 2,4-Diamino-nitrobenzol,
- 3,4-Diamino-nitrobenzol,
- 2,5-Diamino-nitrobenzol,
- 3-Amino-4-hydroxy-nitrobenzol,
- 4-Amino-3-hydroxy-nitrobenzol,
- 5-Amino-2-hydroxy-nitrobenzol,
- 2-Amino-5-hydroxy-nitrobenzol,
- 4-Amino-3-hydroxy-nitrobenzol,
- 5-Amino-2-hydroxy-nitrobenzol,
- 2-Amino-3-hydroxy-nitrobenzol,
- 2-Amino-5-N-(β-hydroxyethyl)amino-nitrobenzol,
- 2-Amino-5-N,N-bis(β-hydroxyethyl)amino-nitrobenzol,
- 2,5-N,N'-(β-Hydroxyethyl)amino-nitrobenzol,
- 2-N-(β-Hydroxyethyl)amino-5-N,N-bis(β-hydroxyethyl)aminonitrobenzol,
- 2-Amino-5-N-(methyl)amino-nitrobenzol,
- 2-N-(Methyl)amino-5-N,N-bis(β-hydroxyethyl)amino-nitrobenzol,
- 2-N-(Methyl)amino-5-(N-methyl-N-β-hydroxyethyl)amino-nitrobenzol,
- 2,5-N,N'-(β-Hydroxyethyl)amino-nitrobenzol,
- 2-N-(β-Hydroxyethyl)amino-5-hydroxy-nitrobenzol,
- 3-Methoxy-4-N-(β-hydroxyethyl)amino-nitrobenzol,
- 2-N-(Methyl)amino-4-β-hydroxyethyloxy-nitrobenzol,
- 2-Amino-3-methyl-nitrobenzol,
- 2-N-(β-hydroxyethyl)amino-5-amino-nitrobenzol,
- 2-Amino-4-chlor-5-N-(β-hydroxyethyl)amino-nitrobenzol,
- 2-Amino-4-methyl-5-N-(β-hydroxyethyl)amino-nitrobenzol,
- 2-Amino-4-methyl-5-N-(methyl)amino-nitrobenzol,
- 2-N-(β-Hydroxyethyl)amino-5-methoxy-nitrobenzol,
- 2-Ammo-5-β-hydroxyethyloxy-nitrobenzol,
- 2-N-(β-Hydroxyethyl)amino-nitrobenzol,
- 3-Amino-4-N-(β-hydroxyethyl)amino-nitrobenzol,
- 3-β-Hydroxyethyloxy-4-N-(β-hydroxethyl)amino-nitrobenzol,
- 2-N-(Methyl)amino-4-β,γ-dihydroxypropyloxy-nitrobenzol,
- 2-N-(β-Hydroxyethyl)amino-5-β-hydroxyethyloxy-nitrobenzol,
- 2-N-(β-Hydroxyethyl)amino-5-β,γ-dihydroxypropyloxy-nitrobenzol,
- 2-Hydroxy-4-N-(β-hydroxyethyl)amino-nitrobenzol,
- 2-N-(Methyl)amino-4-nethyl-5-amino-nitrobenzol,
- 2-Amino-4-isopropy-5-N-(methy)amino-mitrobenzol,
- 2-N-(Methyl)amino-5-(N-methyl-N-β,γ-dihydroxypropyl)amino-nitrobenzol,
- 3-N-(β-Hydroxyethyl)aimno-4-N-(β-hydroxyethyl)amino-nitrobenzol,
- 2-Amino-4-methyl-5-N-(β,γ-dihydroxypropyl)amino-nitrobenzol,
- 2-Amino-4-methyl-5-hydroxy-nitrobenzol,
- 2-N-(β-Hydroxyethyl)amino-4-N-(β-hydroxyethyl)amino-nitrobenzol,
- 2-Amino-5-N-(β-aminoethyl)amino-nitrobenzol,
- 2-N-(β-Aminoethyl)amino-5-methoxy-nitrobenzol,
- 2-N-(Methyl)amino-5-N-(β-aminoethyl)amino-nitrobenzol,
- 2-N-(β-Aminoethyl)amino-5-N,N-(dimethyl)amino-nitrobenzol,
- 3-Amino-4-N-(β-aminoethyl)amino-nitrobenzol,
- 2-Amino-4-methyl-5-N-(β-aminoethyl)amino-nitrobenzol,
- 2-N-(β-Aminoethyl)amino-5-N,N-bis(β-hydroxyethyl)amino-nitrobenzol,
- 3-β-Aminoethyloxy-4-amino-nitrobenzol,
- 2-N-(Methyl)amino-5-(N-δ-amino-n-butyl)amino-nitrobenzol,
- 2-N-(γ-Amino-n-propyl)amino-5-N,N-(dimethyl)amino-nitrobenzol,
- 3-Methoxy-4-N-(β-aminoethyl)amino-nitrobenzol,
- 2-N-(β-Aminoethyl)amino-5-amino-nitrobenzol,
- 2-Amino-4-chlor-5-N-(β-aminoethyl)amino-nitrobenzol,
- 2-N-(β-Aminoethyl)amino-4-methoxy-nitrobenzol,
- 2-N-(β-Aminoethyl)amino-nitrobenzol,
- 2-N-(β-Aminoethyl)amino-5-N-(β-aminoethyl)amino-nitrobenzol,
- 2-N-(β-Aminoethyl)amino-4-β-hydroxyethyloxy-nitrobenzol,
- 3-β-Hydroxyethyloxy-4-N-(β-aminoethyl)amino-nitrobenzol
- 2-Amino-5-aminoethyloxy-nitrobenzol,
- 3-Hydroxy-4-N-(β-aminoethyl)amino-nitrobenzol
- 2-N-(β-Aminoethyl)amino-5-β-hydroxyethyloxy-nitrobenzol,
- 2-N-(β-Aminoethyl)amino-4-hydroxy-nitrobenzol,
- 2-[2-Hydroxy-3-N-(β-hydroxyethyl)amino-6-nitro-benzyloxy]-ethylamin, und
- 2-[2-Hydroxy-3-N-(β-hydroxypropyl)amino-6-nitro-benzyloxy]-ethylamin.

7. Zusammensetzung nach Anspruch 6, **dadurch gekennzeichnet, dass** die nitrierten Benzolfarbstoffe der Formel (IV) unter den folgenden Verbindungen ausgewählt sind:
- 2-Amino-4-methyl-5-N-(β-hydroxyethyl)amino-nitrobenzol,
- 4-N-(β-Ureidoethyl)amino-nitrobenzol,
- 4-(N-Ethyl-N-β-hydroxyethyl)amino-1-N-(β-hydroxyethyl)-amino-nitrobenzol,
- 2-N-(β-Hydroxyethyl)amino-5-methyl-nitrobenzol,
- 5-Chlor-3-N-(ethyl)amino-4-hydroxy-nitrobenzol,
- 5-Amino-3-chlor-4-hydroxy-nitrobenznol,
- 2-N-(γ-Hydroxypropyl)amino-5-N,N-bis(β-hydroxyethyl)amino-nitrobenzol,
- 5-Hydroxy-2-N-(γ-hydroxypropyl)amino-nitrobenzol,
- 1,3-Bis(β-hydyoxyethyl)amino-4-chlor-6-nitrobenzol,
- 3,4-Diamino-ntrobenzol,
- 2-Amino-5-hydroxy-nitrobenzol,
- 2-Amino-3-hydroxy-nitrobenzo, ,
- 2-Amino-5-N-(β-hydroxyethyl)amino-nitrobenzol,
- 2-Amino-5-N,N-bis(β-hydroxyethyl)amino-nitrobenzol,
- 2-N-(β-Hydroxyethyl)amino-5-N,N-bis(β-hydroxyethy)amino-nitrobenzol
- 2-N-(β-Hydroxyethyl)amino-5-hydroxy-nitrobenzol,
- 2-N-(β-Hydroxyethyl)amino-5-amino-nitrobenzol,
- 2-N-(β-Ammoethyl)amino-4-methoxy-nitrobenzol, und
- 2-N-(β-Animoethyl)amino-5-β-hydroxyethyloxy-nitrobenzol.

8. Zusammensetzung nach einem der vorhergehenden Ansprüche, **dadurch gekennzeichnet, dass** der nitrierte Benzolfarbstoff oder die nitrierten Benzolfarbstoffe 0,0005 bis 15 Gew.-% der gebrauchsfertigen Farbmittelzusammensetzung ausmachen.

9. Zusammensetzung nach Anspruch 8, **dadurch gekennzeichnet, dass** der nitrierte Benzolfarbstoff oder die nitrierten Benzolfarbstoffe 0,005 bis 10 Gew.-% der gebrauchsfertigen Farbmittelzusammensetzung ausmachen.

10. Zusammensetzung nach einem der vorhergehenden Ansprüche, **dadurch gekennzeichnet, dass** sie eine oder mehrere oxidationsbasen, die unter den p-Phenylendiammen, p-Aminophenolen, o-Phenylendiaminen und heterocyclischen Basen ausgewählt sind, und/oder einen oder mehrere Koppler enthält, die unter den m-Phenylendiaminen, m-Aminophenolen, m-Dihydroxybenzolen, heterocyclichen Kupplern, wie Indolderivaten, Indolinderivaten, Benzimidazolderivaten, Benzomorpholinderivaten, Sesamolderivaten, Pyridinderivaten, Pyridinderivaten und Pyrazolderivaten, und deren Additionssalzen mit einer Säure ausgewählt sind.

11. Zusammensetzung nach Anspruch 10, **dadurch gekennzeichnet, dass** die Oxidationsbase(n) 0,0005 bis 12 Gew.-% des Gesamtgewichts der gebrauchsfertigen Farbmittelzusammensetzung und der oder die Kuppler 0,0001 bis 10 Gew.-% des Gesamtgewichts der gebrauchsfertigen Farbmittelzusammensetzung ausmachen.

12. Zusammensetzung nach Anspruch 11, **dadurch gekennzeichnet, dass** die Oxidationsbase(n) 0,005 bis 8 Gew.-% des Gesamtgewichts der gebrauchsfertigen Farbmittelzusammensetzung und der oder die Kuppler 0,005 bis 5 Gew.-% des Gesamtgewichts der gebrauchsfertigen Farbmittelzusammensetzung ausmachen.

13. Zusammensetzung nach einem der Ansprüche 10 bis 12, **dadurch gekennzeichnet, dass** die Additionssalze mit einer Säure unter den Hydrochloriden, Hydrobromiden, Sulfaten, Tartraten, Lactaten und Acetaten ausgewählt sind.

14. Zusammensetzung nach einem der Ansprüche 10 bis 13, **dadurch gekennzeichnet, dass** sie mindestens ein Oxidationsmittel enthält.

15. Zusammensetzung nach Anspruch 14, **dadurch gekennzeichnet, dass** das Oxidationsmittel unter Wasserstoffperoxid, Harnstoff peroxid, Alkalimetallbromaten, Salzen von Persäuren, wie Perboraten und Persulfaten, und Enzymen ausgewählt ist.

16. Zusammensetzung nach Anspruch 15, **dadurch gekennzeichnet, dass** die Enzyme unter den Peroxidasen und den Oxidoreduktasen (zwei Elektronen) ausgewählt sind.

17. Zusammensetzung nach Anspruch 16, **dadurch gekennzeichnet, dass** die Oxidoreduktasen (zwei Elektronen) unter den Pyranoseoxidasen, Glucoseoxidasen, Glycerinoxidasen, Lactatoxidasen, Pyruvatoxidasen und Uricasen ausgewählt sind.

18. Zusammensetzung nach Anspruch 16 oder 17, **dadurch gekennzeichnet, dass** die Oxidoreduktasen (2 Elektronen) unter den Uricasen tierischer, mikrobiologischer oder blotechnologischer Herkunft ausgewählt sind.

19. Zusammensetzungnach einem der Ansprüche 16 bis 18, **dadurch gekennzeichnet, dass** die Oxidoreduktase(n) (2 Elektronen) 0,01 bis 20 Gew.-% des Gesamtgewichts der gebrauchsfertigen Farbmittelzusammensetzung ausmachen.

20. Zusammensetzung nach Anspruch 19, **dadurch gekennzeichnet, dass** die Oxidoreduktase(n) (2 Elektronen) 0,1 bis 5 Gew.-% des Gesamtgewichts der gebrauchsfertigen Farbmittelzusamensetzung ausmachen.

21. Zusammensetzung nach einem der Ansprüche 18 bis 20, **dadurch gekennzeichnet, dass** der Donor (oder das Substrat) für die Oxidoreduktase (2 Elektronen) unter Harnsäure und ihren Salzen ausgewählt ist.

22. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

23. Zusammensetzung nach einem der vorhergehenden Ansprüche, **dadurch gekennzeichnet, dass** sie einen pH-Wert im Bereich von 5 bis 11 aufweist.

24. Verfahren zum Färben von Keratinfasern und insbesondere menschlichen Keratinfasern, wie Haaren, **dadurch gekennzeichnet, dass** auf die Fasern mindestens eine gebrauchsfertige Farbmittelzusammensetzung nach einem der vorhergehender Ansprüche während einer Zeitspanne, die zur Bildung der gewünschten Färbung ausreichend ist, aufgebracht wird.

25. Verfahren nach Anspruch 24, **dadurch gekennzeichnet, dass** es einen vorbereitenden Schritt umfasst, der darin besteht, einerseits eine Zusammensetzung (A), die in einem zum Färben geeigneten Medium mindestens einen in den Ansprüchen 1 bis 4 definierten Direktfarbstoff, mindestens einen Direktfarbstoff vom nitrierten Benzoltyp und mindestens eine Oxidationsbase und/oder mindestens einen Kuppler enthält, und andererseits eine Zusammensetzung (B) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird.

26. Vorrichtung mit mehreren Abteilungen oder "Kit" zum Färben mit mehreren Abteilungen, **dadurch gekennzeichnet, dass** eine erste Abteilung die wie in Anspruch 25 definierte Zusammensetzung (A) und eine andere Abteilung die wie in Anspruch 25 definierte Zusammensetzung (B) enthält.
